(19) Europäisches Patentamt
European Patent Office
Office européen des brevets

(11) Veröffentlichungsnummer: **0 226 544**
**B1**

(12) # EUROPÄISCHE PATENTSCHRIFT

(45) Veröffentlichungstag der Patentschrift: **07.03.90**

(21) Anmeldenummer: **86810579.2**

(22) Anmeldetag: **10.12.86**

(51) Int. Cl.[4]: **C07C 233/57,** C07C 233/58, C07C 61/35, C07C 69/753, C07C 47/45, C07C 255/45

(54) **Allyl-bicyclo[2.2.1]hept-5-en-2-carbonsäure(derivate).**

(30) Priorität: **16.12.85 CH 5340/85**

(43) Veröffentlichungstag der Anmeldung: **24.06.87 Patentblatt 87/26**

(45) Bekanntmachung des Hinweises auf die Patenterteilung: **07.03.90 Patentblatt 90/10**

(84) Benannte Vertragsstaaten: **CH DE FR GB IT LI NL**

(56) Entgegenhaltungen:
**EP-A- 0 105 024**
**EP-A- 0 150 015**
**US-A- 4 331 570**

(73) Patentinhaber: **CIBA-GEIGY AG, Klybeckstrasse 141, CH-4002 Basel(CH)**

(72) Erfinder: **Kramer, Andreas, Dr., Bundtels, CH-3186 Düdingen(CH)**
Erfinder: **Darms, Roland, Dr., Kleinfeldweg 3, CH-4106 Therwil(CH)**

EP 0 226 544 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Allyl-bicyclo[2.2.1]hept-5-en-2-carbonsäure(derivate), insbesondere die Carbonsäureamide, ein Verfahren zur Herstellung der Carbonsäureamide, Polymere, die sich von diesen Carbonsäureamiden ableiten, sowie die Verwendung der Carbonsäureamide zur Herstellung gehärteter Produkte.

Derivate der Allyl-bicyclo[2.2.1]hept-5-en-2,3-dicarbonsäure sind bekannt. So beschreibt die EP-A 105,024 Imide dieser Dicarbonsäure sowie die Polymeren, die durch thermische Polymerisation der Monomeren gewonnen werden können. Die polymeren Produkte eignen sich z.B. als Matrixharze für Verbundwerkstoffe oder als Isoliermaterialien.

Derivate der Allyl-bicyclo[2.2.1]hept-5-en-2-carbonsäure sind von A.G. Gonzalez et al. in Tetrahedron Lett. 25(25), 2697-2700 (1984) beschrieben worden. Diese Verbindungen besitzen neben der Allyl- und Carbonsäurefunktion noch eine Hydroxylgruppe in 7-Position. Die Arbeit betrifft die Synthese stereospezifisch funktionalisierter Derivate des Bicyclo[2.2.1]hepten Systems. Eine Anwendungsmöglichkeit für diese Verbindungen oder eine Weiterverarbeitung zu Polymeren ist nicht erwähnt.

Die vorliegende Erfindung betrifft Verbindungen der Formel I

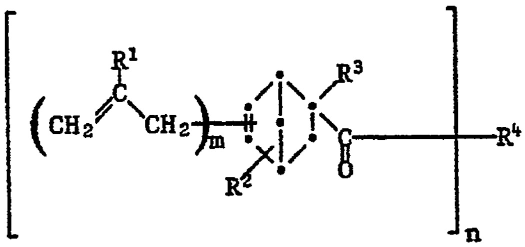 (I),

worin $R^1$, $R^2$ und $R^3$ unabhängig voneinander Wasserstoff oder Methyl bedeuten,
m eine ganze Zahl von 1 bis 5 ist,
n eine ganze Zahl von 1 bis 5 bedeutet, und
$R^4$ $-NH_2$ oder ein n-wertiger Rest eines primären und/oder sekundären aliphatischen, cycloaliphatischen, aromatischen, araliphatischen oder heterocyclischen (Poly)amins nach dem Entfernen von n Aminwasserstoffatomen ist, der über die Aminogruppen an die jeweiligen Carbonylreste gebunden ist.

$R_4$ ist $-NH_2$ oder ein ein- bis fünfwertiger Rest, der sich von (Poly)aminen mit ein bis fünf sekundären und/oder primären Aminogruppen ableitet.
Diese Aminogruppen können Teil eines aliphatischen oder heterocyclischen Systems sein oder sie sind als Substituenten an einen ein- bis fünfwertigen Rest gebunden.
Beispiele für Amine des letzterwähnten Typs sind Verbindungen der Formel II

$$R^6 \!-\!\!(NHR^5)_n \quad (II),$$

worin n eine ganze Zahl von 1 bis 5 ist, $R^5$ Wasserstoff, $C_1$-$C_{20}$-Alkyl, $C_3$-$C_{12}$Alkenylmethyl, $C_3$-$C_{12}$Alkinylmethyl, $C_3$-$C_{12}$Cycloalkyl, Phenyl, Naphthyl, $C_7$-$C_9$Aralkyl oder $C_7$-$C_9$Alkaryl bedeutet und $R^6$ ein n-wertiger aliphatischer, cycloaliphatischer, aromatischer, araliphatischer oder heterocyclischer Rest ist, wobei die Bedeutung der einzelnen Reste $R^5$ eines Moleküls innerhalb der gegebenen Definitionen unterschiedlich sein kann.

Ist $R^5$ $C_1$-$C_{20}$Alkyl, so handelt es sich dabei um einen Rest mit gerader oder mit verzweigter, bevorzugt jedoch mit gerader, Alkylkette. Beispiele für solche Reste sind Methyl, Ethyl, n-Propyl, Isopropyl, n-Butyl, sek.Butyl, Pentyl, Hexyl, 2-Ethylhexyl, Octyl, Decyl, Dodecyl, Tetradecyl, Hexadecyl, Octadecyl oder Eicosyl.

$R_5$ kann auch substituiertes Alkyl sein. Beispiele für Substituenten sind $C_1$-$C_4$Alkoxy, Halogen, insbesondere Chlor oder Brom, oder Cyan.

$R^5$ ist als Alkyl vorzugsweise $C_1$-$C_8$Alkyl, insbesondere jedoch Methyl.

Als $C_3$-$C_{12}$Cycloalkyl bedeutet $R^5$ beispielsweise Cyclopropyl, Cyclobutyl, Cyclopentyl, Cyclohexyl, Cycloheptyl, Cyclooctyl, Cyclodecyl oder Cyclododecyl, besonders bevorzugt jedoch Cyclohexyl.

Als $C_3$-$C_{12}$Alkenylmethyl bedeutet $R^5$ beispielsweise Prop-2-enyl, But-3-enyl, Hex-5-enyl, Oct-7-enyl, Dec-9-enyl oder Dodec-11-enyl. Besonders bevorzugt ist $R^5$ Prop-2-enyl.

Als $C_3$-$C_{12}$Alkinylmethyl bedeutet $R^5$ beispielsweise Prop-2-inyl, But-3-inyl, Hex-5-inyl, Oct-7-inyl, Dec-9-inyl oder Dodec-11-inyl. Besonders bevorzugt ist $R^5$ Propargyl.

Als $C_7$-$C_9$Aralkyl ist $R^5$ beispielsweise Benzyl, α-Methylbenzyl oder α,α-Dimethylbenzyl. Bevorzugt wird Benzyl.

Als $C_7$-$C_9$Alkaryl ist $R^5$ beispielsweise Tolyl oder Xylyl, bevorzugt jedoch Tolyl.

Bevorzugt ist $R^4$ der Rest eines primären oder sekundären Monoamins der Formel II. $R^6$ besitzt in diesem Falle beispielsweise eine der für $R^5$ definierten Bedeutungen oder ist ein fünf- oder sechsgliedriger heterocyclischer Rest mit ein bis drei Sauerstoff-, Schwefel- und/oder Stickstoffatomen.

Beispiele dafür sind Furan-2-yl, Thiophen-2-yl, Pyrrol-2-yl, Pyridin-2-yl, 2-H-Pyran-3-yl, Imidazol-2-yl, Pyrazol-3-yl, Pyrazin-2-yl, Pyrimidin-4-yl, Pyrazin-4-yl, Isothiazol-3-yl, Isoxazol-3-yl, Furazan-3-yl, Pyrrolin-2-yl, Pyrrolidin-2-yl, Imidazolin-2-yl, Pyrazolidin-3-yl, Piperidin-2-yl, Piperazin-2-yl oder Morpholin-2-yl. Besonders bevorzugt wird Furan-2-yl.

Vorzugsweise leitet sich $R^4$ von einem diprimären Diamin oder von einem sekundär-primären Diamin der Formel II ab.

$R^6$ ist dann beispielsweise eine $C_2$-$C_{18}$Alkylenkette, die gegebenenfalls durch einzelne Sauerstoffatome unterbrochen sein kann.

Beispiele für solche Reste sind Ethylen, Trimethylen, Tetramethylen, Pentamethylen, Hexamethylen, Heptamethylen, Octamethylen, Decamethylen, Dodecamethylen, Tetradecamethylen, Hexadecamethylen, Octadecamethylen oder Reste der Formel $-(CH_2-CH_2-O)_s-CH_2-CH_2-$, worin s eine ganze Zahl von 1 bis 8 bedeutet.

Leitet sich $R^4$ von einem cycloaliphatischen Diamin ab, so handelt es sich dabei vorzugsweise um Derivate des Cyclopentan- oder Cyclohexandiamins oder des Bis-(aminocyclohexyl)-methans. Gegebenenfalls sind die Ringe mit ein bis drei Alkylgruppen, vorzugsweise Methyl, substituiert. Beispiele für zweiwertige cycloaliphatische Rest $R^6$ im Amin der Formel II sind Cyclopent-1,4-diyl, Cyclopent-1,3-diyl, Cyclohex-1,2-diyl, Cyclohex-1,3-diyl, Cyclohex-1,4-diyl, Di-cyclohexylmethan-3,3'-diyl, Dicyclohexylmethan-3,4'-diyl, Di-cyclohexylmethan-4,4'-diyl oder Di-3,3'-dimethylcyclohexylmethan-4,4'-diyl. Weitere zweiwertige cycloaliphatische Reste $R^4$ leiten sich von Isophorondiamin, 1,3- und 1,4-(Aminomethyl)-cyclohexan oder 3(4),8(9)-Bis-aminomethyl-tricyclo[5.2.1.$0^{2,6}$]decan ab.

Leitet sich $R^4$ von einem araliphatischen Diamin ab, so handelt es sich dabei beispielsweise um ein Amin der Formel II, worin $R^6$ 1,3- oder 1,4-Xylylen bedeutet.

$R^4$ kann sich auch von einem zweiwertigen aromatischen Diamin ableiten. Dabei handelt es sich um Reste mit mindestens einem aromatischen Ring. Mehrere aromatische Kerne können kondensiert sein oder über ein Brückenglied miteinander verknüpft sein.

Beispiele für zweiwertige aromatische Reste $R^6$ im Amin der Formel II sind 1,3-Phenylen, 1,4-Phenylen, 2,4-Tolylen, 4,4'-Diphenylmethan, 4,4'-Diphenylether, 4,4'-Diphenylsulfon, 4,4'-Diphenylketon, 4,4'-Diphenylsulfid, 4,4'-Diphenyl sowie die entsprechenden 3,3'- oder 3,4'-Derivate.

$R^4$ kann sich auch von einem zweiwertigen heterocyclischen Diamin ableiten. Beispiele für solche Diamine sind 2,4- oder 2,6-Diaminopyridin, 2,4-Diaminopyrimidin, N,N'-Bis-(3-aminopropyl)-piperazin oder 2,4-Diamino-s-triazin.

Sind die Aminogruppen des Restes $R^4$ Teil eines heterocyclischen Systems, so handelt es sich bei diesem Diamin beispielsweise um Piperazin, oder 2-Methylpiperazin oder auch um N-Aminoethylpiperazin.

Beispiele für Amine mit drei bis fünf Aminogruppen, von denen sich $R^4$ ableitet, sind aliphatische Polyamine der Formel $H_2N-(CH_2-CH_2-NH)_t-CH_2-CH_2-NH_2$ (t=1-3), 2,4,6-Triaminocyclohexan, 2,4,6,-Triaminobenzol, 2,4,6,-Triaminomethylbenzol oder 2,4,6-Triamino-s-triazin.

Bevorzugt werden Verbindungen der Formel I, worin $R^1$, $R^2$ und $R^3$ Wasserstoff sind. Der Index m ist bevorzugt 1 oder 2. Ebenfalls bevorzugt werden Verbindungen der Formel I, worin n eine ganze Zahl von 2-5 ist.

Ganz besonders bevorzugt werden Verbindungen der Formel I, worin n eine ganze Zahl von 2 bis 5 ist, und $R^4$ sich ableitet von $\alpha,\omega$-Alkylendiaminen, m- oder p-Phenylendiamin, m- oder p-Xylylendiamin, sowie von Verbindungen der Formeln III, IV, V, VI oder VII

$R^9HN\text{-}(\text{-}Z\text{-}O\text{-})_p\text{-}Z\text{-}NHR^9$ (III),

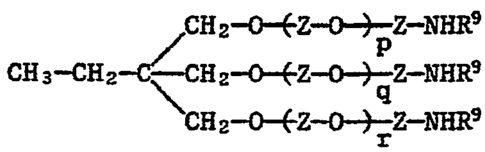 (IV),

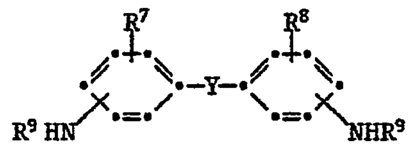 (V),

$R^9HN(\text{-}CH_2\text{-}CH_2\text{-}NH\text{-})_o\text{-}CH_2\text{-}CH_2\text{-}NHR^9$ (VI),

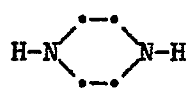 (VII),

worin die Brücke Y eine direkte C-C-Bindung ist, oder -O-, -S-, $-SO_2-$, -CO-, $-CH_2-$ oder $-C(CH_3)_2-$ bedeutet, $R^7$, $R^8$ und $R^9$ unabhängig voneinander Wasserstoff oder $C_1$-$C_5$Alkyl sind, Z eine der Reste $-CH_2-CH_2-$, $-CH_2-CH_2-CH_2-$ oder $-CH_2-CH(CH_3)-$ ist, der index o eine ganze Zahl von 0 bis 2 ist und die Indizes p, q und r unabhängig voneinander ganze Zahlen von 1 bis 8 bedeuten.

Ebenfalls besonders bevorzugt sind Verbindungen der Formel I, worin m 1 oder 2 ist und n 2 bedeutet, $R^4$ ausgewählt wird aus der Gruppe bestehend aus

$-R^{10}N\text{-}(CH_2)_u\text{-}NR^{10}-$, 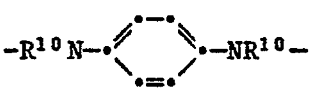, 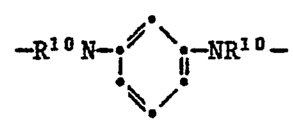 und

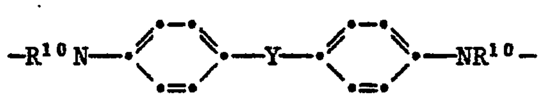 ,

worin Y -O-, $-SO_2-$, -CO- oder $-CH_2-$ ist, $R^{10}$ Wasserstoff, $C_1$-$C_5$Alkyl oder Phenyl ist und u eine ganze Zahl von 2 bis 12 bedeutet.

Von besonderem Interesse sind schliesslich Verbindungen der Formel I, worin $R^1$ und $R^2$ Wasserstoff sind und $R^3$ Methyl darstellt.

Ganz besonders bevorzugt werden die Verbindungen

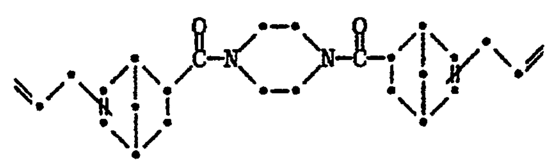

und

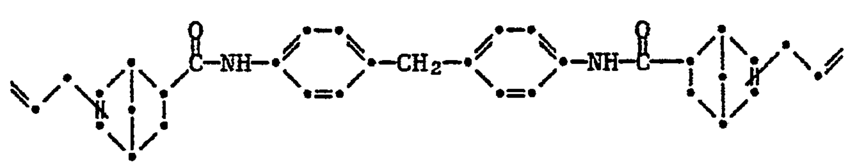

Ebenfalls bevorzugt werden Verbindungen der Formel I, worin m 1 oder 2 ist, n 1 ist, $R^4$ $-NR^{12}R^{13}$ ist und $R^{12}$ und $R^{13}$ unabhängig voneinander Wasserstoff, $C_1$-$C_8$Alkyl, Allyl, Cyclohexyl, Phenyl, Benzyl oder Tolyl bedeuten.

Die Verbindungen der Formel I lassen sich in an sich bekannter Weise durch Amidierung aus den Carbonsäure(ester)n oder Carbonsäurehalogeniden der Formel VIII erhalten. Die Zwischenprodukte der Formel VIII sind neu und stellen ebenfalls einen Gegenstand der Erfindung dar.

Die vorliegende Erfindung betrifft daher auch Verbindungen der Formel VIII

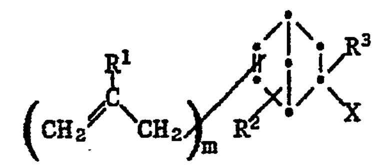

(VIII),

worin $R^1$, $R^2$ und $R^3$ unabhängig voneinander Wasserstoff oder Methyl bedeuten,
X - COOH, $-COOR^{11}$, -COCl, -COBr, -CHO oder -CN ist,
$R^{11}$ $C_1$-$C_{20}$Alkyl, $C_3$-$C_{12}$Alkenylmethyl, $C_3$-$C_{12}$Alkinylmethyl, $C_3$-$C_{12}$Cycloalkyl, Phenyl, Naphthyl, $C_7$-$C_9$Aralkyl, $C_7$-$C_9$Alkaryl oder einen fünf- bis sechsgliedrigen heterocyclischen Ring mit ein bis drei Sauerstoff-, Schwefel- und/oder Stickstoffatomen darstellt und m eine ganze Zahl von 1 bis 5 bedeutet.

Ist $R^{11}$ $C_1$-$C_{20}$Alkyl, $C_3$-$C_{12}$Alkenylmethyl, $C_3$-$C_{12}$Alkinylmethyl, $C_3$-$C_{12}$Cycloalkyl, $C_7$-$C_9$Aralkyl oder $C_7$-$C_9$Alkaryl, so handelt es sich dabei in der Regel um Reste, wie sie weiter oben bereits für die entsprechenden Bedeutungen von $R^5$ definiert wurden.

Stellt $R^{11}$ einen fünf- oder sechsgliedrigen heterocyclischen Ring mit ein bis drei Sauerstoff-, Schwefel- und/oder Stickstoffatomen dar, so handelt es sich dabei um aromatische oder nichtaromatische Reste, wie sie weiter oben bereits für die entsprechend Bedeutungen von $R^6$ definiert wurden.

Besonders bevorzugt werden Verbindungen der Formel VIII, worin X - COOH, $COOR^{11}$, -COCl, bedeutet, $R^{11}$ $C_1$-$C_8$Alkyl, Allyl, Cyclohexyl, Phenyl, Benzyl, Tolyl oder Furan-2-yl darstellt und m 1 oder 2 ist.

Ganz besonders bevorzugt werden Verbindungen der Formel VIII, worin $R^1$, $R^2$ und $R^3$ Wasserstoff sind, X einen der Reste - COOH, $-COOCH_3$, $-COOC_6H_5$ oder -COCl darstellt und m 1 oder 2 bedeutet.

Die Verbindungen der Formel VIII lassen sich in Analogie zum Verfahren, das aus der US-PS 3,105,839 bekannt ist, herstellen. Dabei setzt man gemäss dem nachfolgenden Schema zunächst Cyclopentadien oder Methylcyclopentadien mit einem Allylhalogenid um. Das Reaktionsprodukt wird anschliessend in einer Diels-Alder Reaktion zusammen mit einem Acryl-oder Methacrylsäurederivat zur Verbindung VIII umgesetzt.

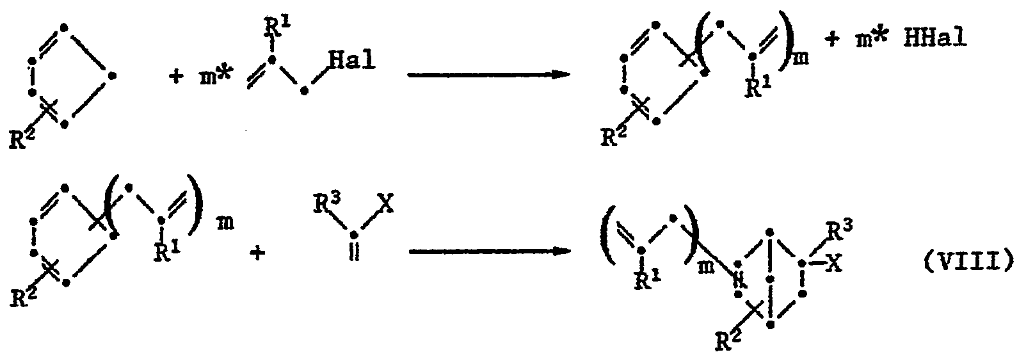


(VIII)

In diesem Schema haben $R^1$, $R^2$, $R^3$, X und m die bereits oben definierte Bedeutung. Als Allylhalogenid setzt man beispielsweise das Chlorid oder das Bromid ein, insbesondere jedoch das Chlorid.

Als Acrylsäure- oder Methacrylsäurederivat verwendet man bevorzugt das Säurechlorid oder den Ester, insbesondere den Methylester.

Die Verbindungen der Formel I können in an sich bekannter Weise z.B. durch Umsetzung einer Carbonsäure, eines Carbonsäureesters oder eines Carbonsäurechlorids bzw. -bromids der Formel VIII mit Ammoniak oder einem (Poly)amin mit primären und/oder sekundären Aminogruppen, beispielsweise mit einem Amin der oben definierten Formel II, hergestellt werden.

Für die Umsetzung mit den Carbonsäurechloriden und -bromiden VIII können primäre oder sekundäre Amine eingesetzt werden, während die Ester der Formel VIII sich mit primären Aminen umsetzen lassen.

Erfolgt die Umsetzung mit einem Säurechlorid oder -bromid, so wird das Ammoniak oder das Amin in der Regel in stöchiometrischen Mengen, bezogen auf die Aminogruppen, eingesetzt. Der freiwerdende Chlorwasserstoff oder Bromwasserstoff wird durch Zugabe stöchiometrischer Mengen einer Base, bevorzugt eines tertiären Amins wie z.B. Triethylamin oder Pyridin, oder durch einen Ueberschuss von Ammoniak oder primärem bzw. sekundärem Amin neutralisiert.

Wünscht man N-substituierte Amide der Formel I, so setzt man sekundäre Amine mit dem Säurechlorid oder -bromid VIII, wie oben beschrieben, um.

Die Umsetzung des Esters VIII erfolgt in der Regel bei erhöhten Temperaturen, bevorzugt bei 120 bis 160°C, mit einem Ueberschuss an primärem Amin II in Gegenwart eines basischen Katalysators, wie beispielsweise einem Alkalialkoholat, insbesondere Natriumethanolat oder einem Alkalihydroxid.
Setzt man den Ester VIII mit Benzylamin um, so lässt sich auch Ammoniumchlorid als Katalysator verwenden.

Gegenstand der Erfindung ist also auch ein Verfahren zur Herstellung von Verbindungen der Formel I durch Umsetzung eines Carbonsäurechlorids der Formel IX

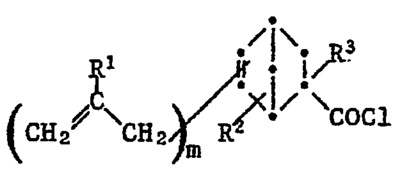

(IX),

worin $R^1$, $R^2$ und $R^3$ unabhängig voneinander Wasserstoff oder Methyl bedeuten und m eine ganze Zahl von 1 bis 5 ist, mit einem primären und/oder sekundären aliphatischen, cycloaliphatischen, aromatischen, araliphatischen oder heterocyclischen (Poly)amin mit ein bis fünf Aminogruppen, wobei die Aminogruppen des (Poly)amins in mindestens der dem Carbonsäurechlorid äquivalenten Menge vorliegen müssen, in Gegenwart von mindestens der dem Carbonsäurechlorid äquivalenten Menge eines tertiären Amins, oder
durch Umsetzung eines Carbonsäurechlorids der Formel IX wie oben definiert, mit mindestens der zweifach äquivalenten Menge an Aminogruppen eines (Poly)amins, wie oben definiert.

Ein weiterer Gegenstand der vorliegenden Erfindung ist ein Verfahren zur Herstellung von Verbindungen der Formel I mit einem aliphatischen, cycloaliphatischen oder araliphatischen Rest $R^4$ durch Umsetzung eines Carbonsäureesters der Formel X

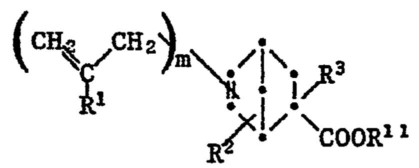

(X),

worin $R^1$, $R^2$, $R^3$, $R^{11}$ und m die bereits weiter oben definierte Bedeutung besitzen, mit einem primären aliphatischen, cycloaliphatischen oder araliphatischen (Poly)amin mit ein bis fünf Aminogruppen, wobei die Aminogruppen des (Poly)amins in etwa der 1,1-bis 2,0-fachen molaren Menge, bezogen auf die Menge des Carbonsäureesters X, vorliegen, in Gegenwart eines basischen Katalysators bei Temperaturen zwischen 120 und 160°C.

Die Umsetzungen können ohne Lösungsmittel oder in Gegenwart eines inerten Lösungsmittels erfolgen. Beispiele für Lösungsmittel sind Kohlenwasserstoffe oder chlorierte Kohlenwasserstoffe, wie Petrolether, Dichlormethan, Chloroform, Benzol, Toluol, Xylol oder Chlorbenzol, sowie Ether, wie Diethylether oder Dioxan.

Weitere Zwischenprodukte der Formel VIII (Aldehyde, Nitrile) können über Zwischenschritte in an sich bekannter Weise in Carbonsäurechloride, -bromide oder Ester umgewandelt werden und dann zu dem entsprechenden Amid der Formel I weiterverarbeitet werden.

Die erfindungsgemässen Amide der Formel I sind flüssige oder niedrigschmelzende feste Stoffe, die sich in der Regel gut in organischen Lösungsmitteln lösen und die zu festen unlöslichen Produkten mit hoher Glasumwandlungstemperatur und Wärme- und Wasserbeständigkeit polymerisiert werden können. Die Polymerisation der Monoamide der Formel I führt in der Regel nur zu oligomeren Produkten, die in organischen Lösungsmitteln löslich sind.

Die Monoamide der Formel I lassen sich aber zusammen mit den anderen Amiden der Formel I als polymerisierbares Lösungsmittel einsetzen. Ganz besonders eignen sich Monoamide mit ethylenisch ungesättigten N-Substituenten, beispielsweise N-Allyl- oder N,N-Diallylmonoamide. Durch die Zugabe von Monoamiden der Formel I zu den höheren Amiden der Formel I lässt sich die Viskosität des Monomergemisches beeinflussen.

Die Monomeren der Formel I (gegebenenfalls gemischt mit Monoamiden I) können direkt verwendet und polymerisiert werden, oder sie können zunächst in einem organischen Lösungunsmittel, wie Toluol, Xylol, Methylethylketon, Glykolether, Aceton oder Dichlormethan, gelöst werden. Diese Lösung kann als Imprägniermittel oder Beschichtungsmittel oder auch als Versandobjekt an den Verbraucher dienen. Die Imprägnierung mit den Monomeren der Formel I kann auch vorteilhafterweise aus der Schmelze erfolgen.

Die erfindungsgemässen monomeren Amide der Formel I zeichnen sich im Vergleich zu entsprechenden Imiden durch eine überraschend hohe Reaktivität aus. So ist eine thermische Polymerisation bereits

bei Temperaturen ab 150°C durchführbar. Ausserdem entstehen bei der Polymerisation keine flüchtigen Komponenten, was für viele Anwendungen, beispielsweise die Herstellung von Lackfilmen oder beim Einsatz als Matrixharze, von Vorteil ist.

Die Erfindung betrifft daher auch Polymere, die dadurch erhältlich sind, dass man eine Verbindung der Formel I, insbesondere solche mit n = 2-5, oder ein Gemisch solcher Verbindungen während 6 bis 60 Stunden, vorzugsweise 6-24 Stunden, auf eine Temperatur zwischen 150 und 300°C, vorzugsweise 150 bis 220°C, ganz besonders bevorzugt 160 bis 220°C erhitzt.

Selbstverständlich können den monomeren Verbindungen inerte und stabile Stoffe, wie Füllmittel, Pigmente, Farbstoffe und andere übliche Additive, zugegeben werden, bevor sie zu vernetzten Gebilden polymerisiert werden.

Die Verbindungen der Formel I können vielseitig angewendet werden, z.B. als Giessharze oder zur Herstellung von hitzebeständigen Klebstoffen, elektrischen Isolierstoffen oder insbesondere auch von Matrixharzen für faserverstärkte Verbundwerkstoffe. Als Verstärkungsmittel eignen sich insbesondere Glas-, Kohlenstoff- und Polyimidfasern.

Die Erfindung betrifft auch die Verwendung der Verbindungen der Formel I zur Herstellung gehärteter Produkte.

<u>Beispiel 1: Herstellung von Allyl-bicyclo[2.2.1]hept-5-en-2-carbonylchlorid</u>

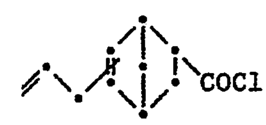

Eine Lösung von 400 g NaOH in 800 ml $H_2O$ und 16 g Benzyltriethylammoniumchlorid (gelöst in 32 ml Ethanol) wird auf 30-35°C erwärmt und unter Rühren innert 15 Minuten mit 264 g Cyclopentadien versetzt. Zur tiefroten klaren Emulsion tropft man innerhalb 45 Minuten 336 g Allylchlorid. Es setzt unmittelbar eine Reaktion ein, die sich durch Wärmeentwicklung und Ausfällung von Natriumchlorid bemerkbar macht. Die Innentemperatur wird mit einem Eisbad zwischen 50° und 55°C gehalten. Sobald das Allylchlorid zugetropft ist wird 30 Minuten bei 50°C nachgerührt. Die Reaktionslösung wird abgekühlt und mit 200 ml Wasser versetzt, dabei geht das ausgefallene Salz in Lösung. Die wässerige Phase wird abgetrennt, die organische Phase zweimal mit gesättigter NaCl-Lösung gewaschen und über Natriumsulfat filtriert. Das nicht umgesetzte Cyclopentadien und Allylchlorid wird bei Raumtemperatur und reduziertem Druck abdestilliert. Diese Reinigungsoperation wird abgebrochen sobald ein absoluter Druck von 25 mm erreicht wird. Die Analyse des Rückstandes durch Chromatographie zeigt, dass nebst Allylcyclopentadien (ca. 75 %) als Nebenprodukte noch Di- und Triallylcyclopentadien, Dicyclopentadien und Diallyldicyclopentadien vorhanden sind 360 g rohes Allylcyclopentadien werden in 400 ml Methylenchlorid aufgenommen. Zu dieser klaren rotbraunen Lösung tropft man innerhalb einer Stunde bei 20°-25°C unter Eiskühlung 252 g Acrylsäurechlorid und rührt anschliessend 2 Stunden bei 20°C. Die Reaktionslösung wird am Rotationsverdampfer eingeengt und der Rückstand bei einem absoluten Druck von 20 mm destilliert. Zwischen 112°C und 120°C gehen 377 g Allyl-bicyclo[2.2.1]hept-5-en-2-carbonylchlorid über, was einer Ausbeute von 68 % der Theorie entspricht. Die gelbe Flüssigkeit kann unter Stickstoff längere Zeit bei 0°C gelagert werden. Zur Charakterisierung wird das Säurechlorid in den entsprechenden Methylester übergeführt. Die gaschromatographischen Daten stimmen mit dem in Beispiel 5 hergestellten Methylester überein.

| Analyse (Methylester) | | |
|---|---|---|
| | % C | % H |
| berechnet für $C_{12}H_{16}O_2$ | 74,97 | 8,39 |
| gefunden | 73,95 | 8,35 |

<u>Beispiel 2: Herstellung von Allyl-bicyclo[2.2.1]hept-5-en-2-methyl-2-carbonylchlorid</u>

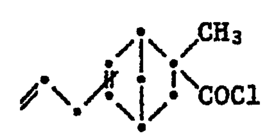

Verwendet man anstelle der in Beispiel 1 eingesetzten Acrylverbindung die entsprechende Methacrylverbindung und geht im übrigen in gleicher Weise wie in Beispiel 1 beschrieben vor, so erhält man das oben genannte Säurechlorid. Die Verbindung wird über den Methylester (Beispiel 6) charakterisiert.

Beispiel 3: Herstellung von Methallyl-methyl-bicyclo[2.2.1]hept-5-en-2-carbonylchlorid bzw. -2-methylester

a) 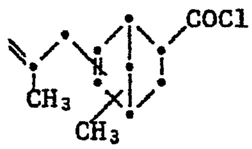 b) 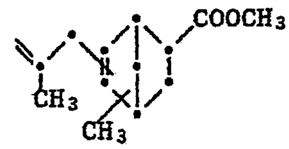

a) Ersetzt man Cyclopentadien und Allylchlorid durch Methylcyclopentadien und Methallylchlorid und geht im übrigen in gleicher Weise wie in Beispiel 1 beschrieben vor, so erhält man das oben genannte Säurechlorid.

Aur Charakterisierung wird das Säurechlorid in den Methylester übergeführt und mit dem Produkt verglichen, das gemäss Syntheseweg b) erhalten wird. Beide Verbindungen stimmen in ihren gaschromatographischen Daten überein.

b) Zur einer Lösung von 24,6 g Methallyl-methylcyclopentadien, hergestellt aus Methallylchlorid und Methylcyclopentadien gemäss Beispiel 1, in 100 ml Ether werden innerhalb 20 Minuten 12 g Acrylsäuremethylester, gelöst in 50 ml Ether, zugetropft. Die klare Reaktionslösung wird über Nacht am Rückfluss gekocht. Der Ether wird abdestilliert und der Rückstand über eine Kolonne fraktioniert. Bei einem Druck von 20 mm destillieren zwischen 122° bis 127°C 13,5 g (51 % der Theorie) Ester als schwach gelbe Flüssigkeit; $n_D^{20}$ = 1,4843.

Analyse

| | % C | % H |
|---|---|---|
| berechnet für $C_{14}H_{20}O_2$ | 76,33 | 9,15 |
| gefunden | 76,91 | 9,20 |

Beispiel 4: Herstellung von Diallyl-bicyclo[2.2.1]hept-5-en-2-carbonylchlorid

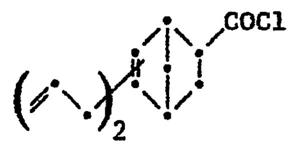

Unter den gleichen Reaktionsbedingungen wie in Beispiel 1 tropft man zu 300 g NaOH in 600 ml $H_2O$ und 12 g Benzyltriaethylammoniumchlorid (gelöst in 24 ml Ethanol), 132 g Cyclopentadien und 321 g Allylchlorid. Nach den Zutropfen rührt man 2 Stunden bei 65°C. Das gebildete Salz wird durch Zusatz von Wasser aufgelöst und die Phasen getrennt. Die organische Phase wird zweimal mit gesättigter NaCl-Lösung gewaschen und über Natriumsulfat filtriert. Die Destillation des rotbraunen Filtrates im Vakuum ergibt 135 g Diallylcyclopentadien; Sdp: 70-82°C bei 20 mm.

Analyse (Diallylcyclopentadien)

| | % C | % H |
|---|---|---|
| berechnet für $C_{11}H_{14}$ | 90,35 | 9,65 |
| gefunden | 90,18 | 9,61 |

81 g Diallylcyclopentadien werden in 200 ml Methylenchlorid gelöst. Zu dieser Lösung tropft man innerhalb 30 Minuten bei 20°C unter Eiskühlung 45 g Acrylsäurechlorid und rührt anschliessend eine Stunde bei Raumtemperatur. Die Destillation des Reaktionsproduktes ergibt 84,4 g (73 % der Theorie) Diallyl-bicyclo[2.2.1]hept-5-en-2-carbonylchlorid; Sdp.= 125-134°C bei 20 mm.

Analyse

| | % Cl |
|---|---|
| berechnet für $C_{14}H_{17}OCl$ | 14,98 |
| gefunden | 14,10 |

Beispiel 5: Herstellung von Allyl-bicyclo[2.2.1]hept-5-en-2-carbonsäuremethylester

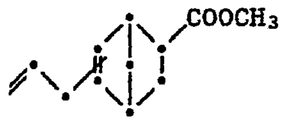

Zu einer Lösung von 170 g Allylcyclopentadien, hergestellt gemäss Beispiel 1, in 200 ml Ether werden innerhalb 30 Minuten 137 g Acrylsäuremethylester zugetropft. Die klare Lösung wird zwei Stunden am Rückfluss gekocht. Der Ether wird abdestilliert und der Rückstand im Vakuum über eine Kolonne fraktioniert. Bei einem Druck von 20 mm destillieren zwischen 107° und 115°C 163 g Allyl-bicyclo[2.2.1]-hept-5-en-2-carbonsäuremethylester als schwach gelbe Flüssigkeit; $n_D^{20}$ = 1,4863.

| Analyse | % C | % H |
|---|---|---|
| berechnet für $C_{12}H_{16}O_2$ | 74,97 | 8,39 |
| gefunden | 74,93 | 8,38 |

Beispiel 6: Herstellung von Allyl-bicyclo[2.2.1]hept-5-en-2-methyl-2-carbonsäuremethylester

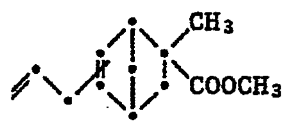

Verwendet man anstelle der in Beispiel 5 eingesetzten Acrylverbindung die entsprechende Methacrylverbindung und geht im übrigen in gleicher Weise wie in Beispiel 5 beschrieben vor, so erhält man das oben genannte Produkt; $n_D^{20}$ = 1,4810.

| Analyse | % C | % H |
|---|---|---|
| berechnet für $C_{13}H_{18}O_2$ | 75,69 | 8,80 |
| gefunden | 76,00 | 8,70 |

Herstellung der Allyl-bicyclo[2.2.1]hept-5-en-2-carbonsäureamide und -diamide

Verfahren A: via Säurechlorid

0,11 Mol Monoamin (0,055 Mol Diamin) gelöst in 25 ml Methylenchlorid werden bei 10° bis 25°C im Verlauf von 30 Minuten zu einer Lösung von 0,1 Mol Allyl-bicyclo[2.2.1]hept-5-en-2-carbonylchlorid und 0,12 Mol Pyridin zugetopft. Dann rührt man 3 Stunden bei 20°C, versetzt mit 200 ml Wasser und trennt die Phasen. Die organische Phase wird mit 1 N HCl, wässeriger Sodalösung und gesättigter Natriumchloridlösung gewaschen, über Natriumsulfat getrocknet, filtriert und das Lösungsmittel bei 60°C am Rotationsverdampfer abdestilliert. Der Rückstand wird 2 Stunden im Vakuum bei 120° bis 160°C gerührt.

Verfahren B: via Methylester

Eine Mischung aus 0,105 Mol Allyl-bicyclo[2.2.1]hept-5-en-2-carbonsäuremethylester, 0,1 Mol eines aliphatischen Amins (0,05 Mol Diamin) und 50 mg Natriumethylat wird im Verlauf von 16 Stunden unter Rühren am absteigenden Kühler auf 160°C erhitzt. Danach wird der Druck auf 0,1 mm reduziert und die Mischung zwei Stunden bei 160°C gerührt.

Beispiel 7: Herstellung von Allyl-bicyclo[2.2.1]hept-5-en-2-carbonsäureamid

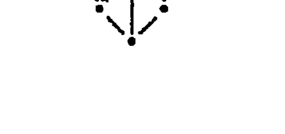

Zu 50 ml 25 %igen wässerigen Ammoniaks tropft man bei 0°C innerhalb 10 Minuten 9,8 g Allyl-bicyclo[2.2.1]hept-5-en-2-carbonylchlorid. Man rührt 15 Minuten bei Raumtemperatur nach und dekantiert das Wasser ab. Der Rückstand wird in 50 ml Ether gelöst, mit gesättigter Soda- und Natriumchloridlösung gewaschen, über Natriumsulfat getrocknet und eingeengt. Man erhält 6,5 g (73 % der Theorie) eines weissen Feststoffs. Zur Reinigung wird aus Wasser umkristallisiert; Smp.= 120-122°C.

| Analyse | | | |
|---|---|---|---|
| | % C | % H | % N |
| berechnet für $C_{11}H_{15}NO$ | 74,54 | 8,53 | 7,90 |
| gefunden | 73,41 | 8,41 | 7,75 |

Beispiel 8: Herstellung von Allyl-bicyclo[2.2.1]hept-5-en-2-carbonsäure-N-methylamid

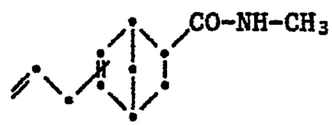

Zu 50 ml einer 40 %igen wässerigen Methylaminlösung tropft man unter Kühlung 9,8 g Allyl-bicyclo[2.2.1]hept-5-en-2-carbonylchlorid. Nach einer Stunde bei 20°C versetzt man mit 100 ml Ether. Die organische Phase wird abgetrennt und wie in Beispiel 4 beschrieben aufgearbeitet. Man erhält 8,0 g (84 % der Theorie) einer gelben Flüssigkeit. Zur Charakterisierung wird eine Probe im Kugelrohr destilliert; $n_D^{20}$ = 1,5160; $\eta_{25}$ = 420 mPas.

| Analyse | | | |
|---|---|---|---|
| | % C | % H | % N |
| berechnet für $C_{12}H_{17}NO$ | 75,35 | 8,96 | 7,32 |
| gefunden | 75,63 | 8,99 | 6,96 |

Beispiel 9: Herstellung von Allyl-bicyclo[2.2.1]hept-5-en-2-carbonsäure-N-allylamid

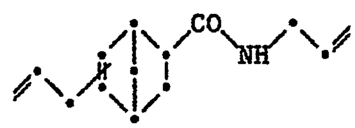

54 g Allylamin und 206 g Allyl-bicyclo[2.2.1]hept-5-en-2-carbonylchlorid werden nach Verfahren A umgesetzt. Die Destillation des Rohproduktes ergibt 182 g (84 % der Theorie) Amid als farblose Flüssigkeit;

Sdp.= 122-128°C bei 0,02 mm; $n_D^{20}$ = 1,5181; $\eta_{25}$ = 217,8 m Pas.

| Analyse | | | |
|---|---|---|---|
| | % C | % H | % N |
| berechnet für $C_{14}H_{19}NO$ | 77,38 | 8,81 | 6,45 |
| gefunden | 76,72 | 8,70 | 6,30 |

Die Polymerisation während 16 Stunden bei 220°C ergibt einen Festkörper mit einer Glasumwandlungstemperatur von 128,5°C.

Beispiel 10: Herstellung von Allyl-bicyclo[2.2.1]hept-5-en-2-carbonsäure-N-cyclohexylamid

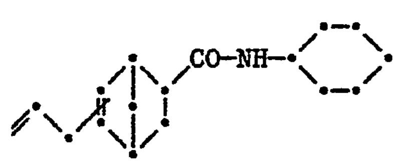

2,8 g Cyclohexylamin und 39,3 g Allyl-bicyclo[2.2.1]hept-5-en-2-carbonylchlorid werden nach Verfahren A umgesetzt. Man erhält 40,7 g (78,9 % der Theorie) eines rotbraunen, flüssigen Harzes mit $\eta_{40}$ = 1128 mPas.

| Analyse | | | |
|---|---|---|---|
| | % C | % H | % N |
| berechnet für $C_{17}H_{24}NO$ | 79,02 | 9,36 | 5,46 |
| gefunden | 78,49 | 9,60 | 5,40 |

Die Polymerisation während 10 Stunden bei 200°C ergibt einen klaren Festkörper mit einer Glasumwandlungstemperatur von 72,5°C. Das Polymer ist gut löslich in Aceton, Methylenchlorid und Tetrahydrofuran. Die Gelpermeationschromatographie (GPC) in Tetrahydrofuran zeigt ein $\bar{M}_n$ von 500 und ein $\bar{M}_w$ von 1363.

Beispiel 11: Herstellung von Allyl-bicyclo[2.2.1]hept-5-en-2-carbonsäure-N-phenylamid

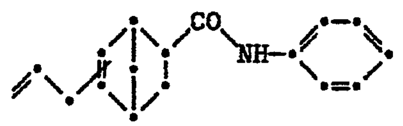

20,5 g Anilin und 39,3 g Allyl-bicyclo-[2.2.1]hept-5-en-2-carbonylchlorid werden nach Verfahren A umgesetzt. Man erhält 40,6 g (80,3 % der Theorie) eines braunen, bei Raumtemperatur festen Harzes mit einem Erweichungspunkt von 66°C. Das IR-Spektrum zeigt eine Bande bei 3300 $cm^{-1}$ (-NH-) und bei 1660 $cm^{-1}$ (>C=O).

Mittels Gelpermeationschromatographie (in THF) wird ein mittleres Molekulargewicht von 308 ($\bar{M}_n$) bzw. 311 ($\bar{M}_w$) gemessen.

| Analyse | | | |
|---|---|---|---|
| | % C | % H | % N |
| berechnet für $C_{17}H_{19}NO$ | 80,60 | 7,56 | 5,53 |
| gefunden | 80,45 | 7,58 | 5,42 |

Die Polymerisation während 10 Stunden bei 200°C ergibt einen braunen Festkörper mit einer Glasumwandlungstemperatur von 94°C. Das Polymere ist gut löslich in Aceton, Methylenchlorid und Tetrahydrofuran. Die Gelpermeationschromatographie (GPC) in Tetrahydrofuran zeight ein $\bar{M}_n$ von 640 und ein $\bar{M}_w$ von 2439.

Beispiel 12: Herstellung von Allyl-bicyclo[2.2.1]hept-5-en-2-carbonsäure-N-furfurylamid

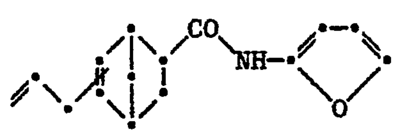

21,4 g Furfurylamin und 39,3 g Allyl-bicyclo[2.2.1]hept-5-en-2-carbonylchlorid werden nach Verfahren A umgesetzt. Man erhält 38,1 g (73,8 % der Theorie) eines rotbraunen, flüssigen Harzes mit $\eta_{25}$ = 1287,2 m Pas.

| Analyse | | | |
|---|---|---|---|
| | % C | % H | % N |
| berechnet für $C_{16}H_{20}NO_2$ | 74,39 | 7,80 | 5,42 |
| gefunden | 74,45 | 7,43 | 5,35 |

Die Polymerisation während 10 Stunden bei 200°C ergibt einen klaren, rotbraunen Festkörper mit einer Glasumwandlungstemperatur von 80°C.

Beispiel 13: Herstellung von Allyl-bicyclo[2.2.1]hept-5-en-2-carbonsäure-N-(2-ethyl)-hexylamid

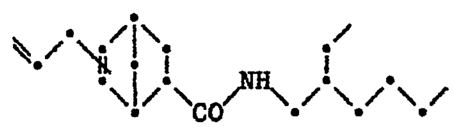

12,9 g 2-Ethyl-hexylamin und 21 g Allyl-bicyclo[2.2.1]hept-5-en-2-carbonsäuremethylester werden nach Verfahren B umgesetzt. Die Destillation des Rohproduktes ergibt 20,6 g (71 % der Theorie) Amid als leicht gelbe Flüssigkeit mit $n_D^{20}$ = 1,4930 und $\eta_{25}$ = 442 m Pas.

| Analyse | | | |
|---|---|---|---|
| | % C | % H | % N |
| berechnet für $C_{19}H_{31}NO$ | 78,84 | 10,80 | 4,84 |
| gefunden | 78,11 | 10,91 | 5,06 |

Beispiel 14: Herstellung von Allyl-bicyclo[2.2.1]hept-5-en-2-carbonsäure-N-benzylamid

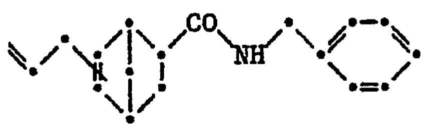

Man mischt 9,6 g Allyl-bicyclo[2.2.1]hept-5-en-2-carbonsäuremethylester mit 30 ml Benzylamin und 1 g Ammoniumchlorid und erhitzt die Mischung 3 Stunden am Rückfluss. Nach dem Abkühlen gibt man 100 ml Methylenchlorid zu und wäscht mit 1 N HCl wässeriger Sodalösung und gesättigter Natriumchloridlösung. Die organische Pase wird getrocknet, filtriert und eingeengt. Man erhält 3,8 g (73,4 % der Theorie) eines roten, flüssigen Harzes. Zur Charakterisierung wird eine Probe im Kugelrohr destilliert. Das Destillat ist ein hellgelbes Oel mit $\eta_{25}$ = 310 mPas.

| Analyse | | | |
|---|---|---|---|
| | % C | % H | % N |
| berechnet für $C_{18}H_{21}NO$ | 80,86 | 7,92 | 5,24 |
| gefunden | 80,89 | 7,97 | 5,59 |

Beispiel 15: Herstellung von Allyl-bicyclo[2.2.1]hept-5-en-2-carbonsäure-N,N'-dimethylamid

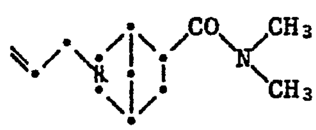

Eine Lösung von 19,6 g Allyl-bicyclo[2.2.1]hept-5-en-2-carbonylchlorid in 200 ml Dioxan wird bei 20°C im Verlauf von 30 Minuten zu 40,9 g Dimethylamin (33 %ig in Ethanol) getropft. Nach einer Stunde Rühren bei 20°C filtriert man das trübe Reaktionsgemisch über Filtrierhilfe und destilliert das Dioxan ab. Der Rückstand wird in 100 ml Methylenchlorid aufgenommen, mit wässeriger Sodalösung und gesättigter Natriumchloridlösung gewaschen, über Natriumsulfat getrocknet, filtriert und eingeengt. Zurück bleiben 18,5 g (90,3 % der Theorie) einer gelben Flüssigkeit. Das Rohprodukt wird bei 140-150°C/0,1 mm destilliert; $n_D^{20}$ = 1,5124.

| Analyse | | | |
|---|---|---|---|
| | % C | % H | % N |
| berechnet für $C_{13}H_{19}NO$ | 76,06 | 9,33 | 6,82 |
| gefunden | 76,59 | 9,37 | 6,40 |

Beispiel 16: Herstellung von Allyl-bicyclo[2.2.1]hept-5-en-2-carbonsäure-N,N'-diallylamid

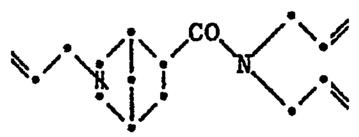

87,5 g Diallylamin und 175 g Allyl-bicyclo[2.2.1]hept-5-en-2-carbonylchlorid werden nach Verfahren A umgesetzt. Die Destillation des Rohproduktes ergibt 173 g (75 % der Theorie) Amid als leicht gelbe Flüssigkeit mit $n_D^{20}$ = 1,5142 und $\eta_{25}$ = 35,5 mPas.

| Analyse | %C | %H | %N |
|---|---|---|---|
| berechnet für $C_{17}H_{23}NO$ | 79,33 | 9,01 | 5,44 |
| gefunden | 78,79 | 8,99 | 5,35 |

Beispiel 17: Herstellung von Allyl-bicyclo[2.2.1]hept-5-en-2-carbonsäure-N,N'-diisobutylamid

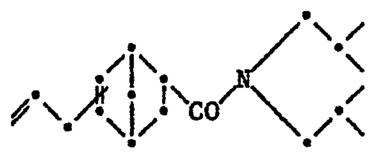

14,2 g Diisobutylamin und 19,6 g Allyl-bicyclo[2.2.1]hept-5-en-2-carbonylchlorid werden nach Verfahren A umgesetzt. Man erhält 22,1 g (76,5 % der Theorie) eines braunen, flüssigen Harzes. Zur Charakterisierung wird eine Probe im Kugelrohr destilliert. Das Destillat ist eine hellgelbe Flüssigkeit mit $n_D^{20}$ = 1,4915 und $\eta_{25}$ = 127 mPas.

| Analyse | %C | %H | %N |
|---|---|---|---|
| berechnet für $C_{19}H_{31}NO$ | 78,84 | 10,80 | 4,84 |
| gefunden | 78,64 | 10,66 | 4,72 |

Beispiel 18: Herstellung von Allyl-bicyclo[2.2.1]hept-5-en-2-carbonsäure-N,N'-diphenylamid

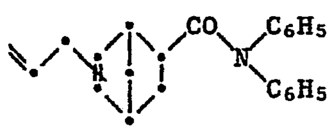

37,2 g Diphenylamin und 39,3 g Allyl-bicyclo[2.2.1]hept-5-en-2-carbonylchlorid werden nach Verfahren A umgesetzt. Man erhält 50,6 g (77 % der Theorie) eines rotbraunen, flüssigen Harzes mit $\eta_{25}$ = 4846 mPas.

| Analyse | %C | %H | %N |
|---|---|---|---|
| berechnet für $C_{23}H_{23}NO$ | 83,85 | 7,04 | 4,25 |
| gefunden | 83,56 | 7,03 | 4,37 |

Die Polymerisation während 10 Stunden bei 200°C ergibt einen klaren, rotbraunen Festkörper mit einer Glasumwandlungstemperatur von 58°C. Die Gelpermeationschromatographie (GPC) in Tetrahydrofuran zeigt ein $\bar{M}_n$ von 476 und ein $\bar{M}_w$ von 1701.

Beispiel 19: Herstellung von N,N'-Ethylen-bis-(allyl-bicyclo[2.2.1]hept-5-en-2-carbonsäureamid

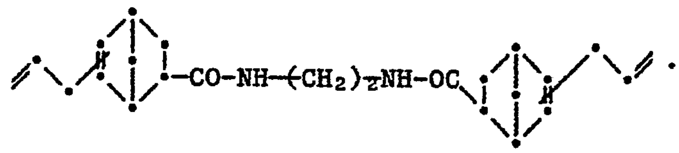

9,0 g Ethylendiamin und 58,9 g Allyl-bicyclo[2.2.1]hept-5-en-2-carbonylchlorid werden nach Verfahren A umgesetzt. Man erhält 47,1 g (82,4 % der Theorie) eines zähflüssigen, rotbraunen Harzes mit $\eta_{80}$ = 2597 mPas.

| Analyse | %C | %H | %N |
|---|---|---|---|
| berechnet für $C_{24}H_{32}N_2O_2$ | 75,75 | 8,48 | 7,36 |
| gefunden | 75,33 | 8,35 | 7,09 |

Beispiel 20: Herstellung von N,N'-Hexamethylen-bis-(allyl-bicyclo[2.2.1]hept-5-en-2-carbonsäureamid

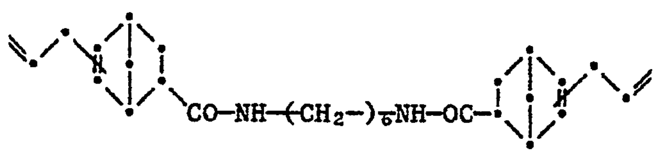

A) 52,3 g 1,6-Diaminohexan und 172 g Allyl-bicyclo[2.2.1]hept-5-en-2-carbonylchlorid werden nach Verfahren A umgesetzt. Man erhält 161,5 g (85 % der Theorie) eines zähflüssigen, roten Harzes mit $\eta_{80}$ = 105 mPas.

| Analyse | %C | %H | %N |
|---|---|---|---|
| berechnet für $C_{28}H_{40}N_2O_2$ | 77,02 | 9,23 | 6,42 |
| gefunden | 77,16 | 9,14 | 5,95 |

B) 55,1 g 1,6-Diaminohexan und 191,7 g Allyl-bicyclo[2.2.1]hept-5-en-2-carbonsäuremethylester werden nach Verfahren B umgesetzt. Man erhält 201 g (97 % der Theorie) eines orangen Harzes mit $\eta_{80}$ = 310 mPas.

| Analyse | %C | %H | %N |
|---|---|---|---|
| berechnet für $C_{28}H_{40}N_2O_2$ | 77,02 | 9,23 | 6,42 |
| gefunden | 76,31 | 9,41 | 6,53 |

Beispiel 21: Herstellung von Bis[4-(allyl-bicyclo[2.2.1]hept-5-en-2-carbonsäureamidophenyl]-methan

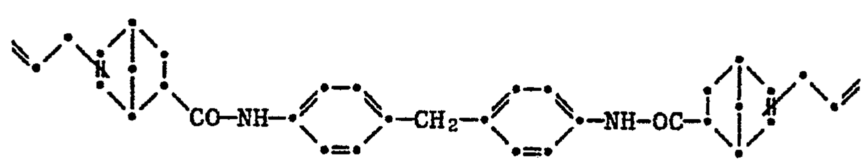

67 g 4,4'-Diaminodiphenylmethan und 127 g Allyl-bicyclo[2.2.1]hept-5-en-2-carbonylchlorid werden nach Verfahren A umgesetzt. Man erhält 157 g (94 % der Theorie) eines braunen Festharzes mit einem Erweichungspunkt von 66°C. Mittels Gelpermeationschromatographie (in THF) misst man ein mittleres Molekulargewicht von 677 ($\bar{M}_n$) bzw. 779 ($\bar{M}_w$). Das IR-Spektrum zeigt eine Absorptionsbande bei 3300 $cm^{-1}$ (-NH-) und 1660 $cm^{-1}$ ($>C=O$).

| Analyse | % C | % H | % N |
|---|---|---|---|
| berechnet für $C_{35}H_{38}N_2O_2$ | 81,05 | 7,38 | 5,40 |
| gefunden | 80,32 | 7,46 | 5,33 |

Beispiel 22: Herstellung von N,N'-m-Xylylen-bis-(allyl-bicyclo[2.2.1]hept-5-en-2-carbonsäureamid

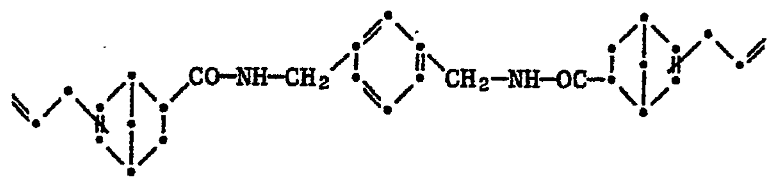

27 g α,α'-Diamino-m-xylol und 78,6 g Allyl-bicyclo[2.2.1]hept-5-en-2-carbonylchlorid werden nach Verfahren A umgesetzt. Man erhält 73 g (80 % der Theorie) eines rotbaunen Festharzes mit einem Erweichungspunkt von 106°C und einem durch Gelpermeationschromatographie (THF) ermittelten Molekulargewicht von 530 ($\bar{M}_n$) bzw. 1441 ($\bar{M}_w$). Das IR-Spektrum zeigt eine Absorptionsbande bei 3300 $cm^{-1}$ (-NH-) und bei 1650 $cm^{-1}$ 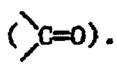.

| Analyse | % C | % H | % N |
|---|---|---|---|
| berechnet für $C_{30}H_{36}N_2O_2$ | 78,91 | 7,95 | 6,13 |
| gefunden | 78,34 | 8,00 | 5,64 |

Beispiel 23: Herstellung von N,N'-4,4'-(3,3'Dimethyl)-diphenyl-bis-(allyl-bicyclo[2.2.1 ]hept-5-en-2-carbonsäureamid)

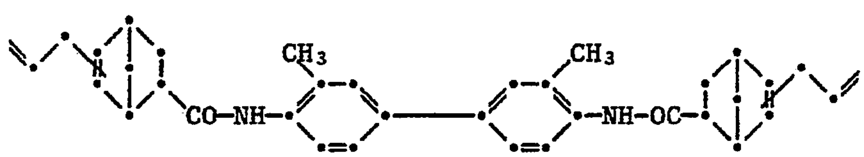

29.0 g o-Tolidin und 58,9 g Allyl-bicyclo[2.2.1]hept-5-en-2-carbonylchlorid werden nach Verfahren A umgesetzt. Man erhält 69,2 g (80 % der Theorie) eines braunen Festharzes mit einem Erweichungspunkt von 82°C und einem durch Gelpermeationschromatographie (THF) ermittelten Molekulargewicht von 739 ($\bar{M}_n$) bzw. 2786 ($\bar{M}_w$). Das IR-Spektrum zeigt eine Absorptionsbande bei 3350 $cm^{-1}$ (-NH-) bzw. bei 1660 $cm^{-1}$ 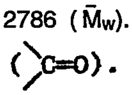.

| Analyse | % C | % H | % N |
|---|---|---|---|
| berechnet für $C_{36}H_{39}N_2O_2$ | 81,32 | 7,39 | 5,27 |
| gefunden | 81,10 | 7,62 | 4,80 |

Beispiel 24: Herstellung von N,N'-Diethylen-bis-(allyl-bicyclo[2.2.1]-hept-5-en-2-carbonsäureamid

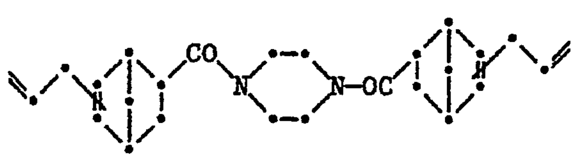

47,3 g Piperazin und 220 g Allyl-bicyclo [2.2.1]-hept-5-en-2-carbonylchlorid werden nach Verfahren

A umgesetzt. Man erhält 206 g (93 % der Theorie) eines braunroten Harzes, das bei Raumtemperatur gerade noch flüssig ist. Durch Gelpermeationschromatographie (THF) ermittelt man ein Molekulargewicht von 403 ($\bar{M}_n$) bzw. von 809 ($\bar{M}_w$). Im IR-Spektrum tritt eine Absorptionsbande bei 1650 $cm^{-1}$ (>C=O).

| Analyse | % C | % H | % N |
|---|---|---|---|
| berechnet für $C_{26}H_{32}N_2O_2$ | 77,19 | 7,97 | 6,92 |
| gefunden | 76,09 | 8,37 | 6,56 |

Beispiel 25: Herstellung von-Bis[4-(diallyl-bicyclo [2.2.1]-hept-5-en-2-carbonsäureamidophenyl]

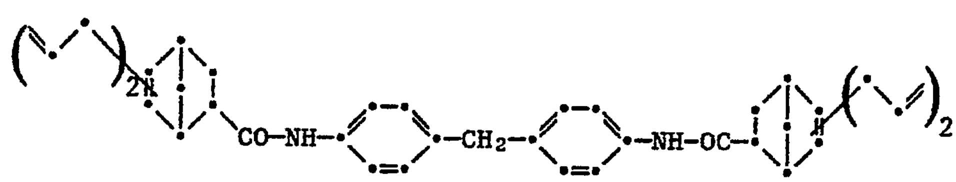

15,7 g 4,4'-Diaminodiphenylmethan und 38 g Diallyl-bicyclo[2.2.1]hept-5-en-2-carbonylchlorid werden nach Verfahren A umgesetzt. Man erhält 35,3 g (74 % der Theorie) eines braunen Festharzes mit einem Erweichungspunkt von 82°C und einem durch Gelpermeationschromatographie (THF) ermittelten Molekulargewicht von 877 ($\bar{M}_n$) bzw. 1501 ($\bar{M}_w$). Im IR-Spektrum findet sich eine Absorptionsbande bei 3310 $cm^{-1}$ (-NH-) und bei 1660 $cm^{-1}$ (>C=O).

| Analyse | % C | % H | % N |
|---|---|---|---|
| berechnet für $C_{41}H_{46}N_2O_2$ | 82,24 | 7,74 | 4,68 |
| gefunden | 81,55 | 7,80 | 4,52 |

Beispiel 26: Herstellung von Bis[4-(methallyl-methyl-bicyclo[2.2.1]hept-5-en-2-carbonsäureamido-phenyl)]-methan

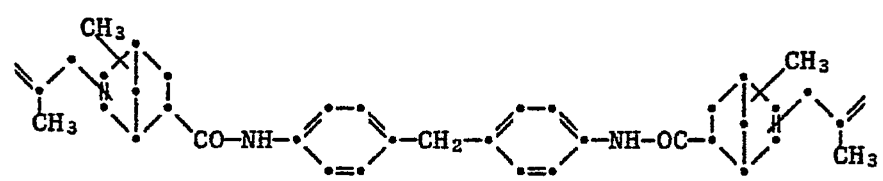

19,8 g 4,4'-Diaminodiphenylmethan und 44,9 g Methallyl-methyl-bicyclo[2.2.1]hept-5-en-2-carbonylchlorid werden nach Verfahren A umgesetzt. Man erhält 45,3 g (79 % der Theorie) eines rotbraunen Festharzes mit einem Erweichungspunkt von 74°C und einem durch Gelpermeationschromatographie (THF) ermittelten Molekulargewicht von 794 ($\bar{M}_n$) bzw. bei 1114 ($\bar{M}_w$). Das IR-Spektrum zeigt eine Absorptionsbande bei 3310 $cm^{-1}$ (-NH-) bzw. 1665 $cm^{-1}$ (>C=O).

| Analyse | % C | % H | % N |
|---|---|---|---|
| berechnet für $C_{39}H_{46}N_2O_2$ | 81,49 | 8,07 | 4,57 |
| gefunden | 80,85 | 8,00 | 4,58 |

Beispiel 27: Herstellung von Bis[4-(allyl-bicyclo [2.2.1]-hept-5-en-2-methyl-2-carbonsäureamido-phenyl)]-methan

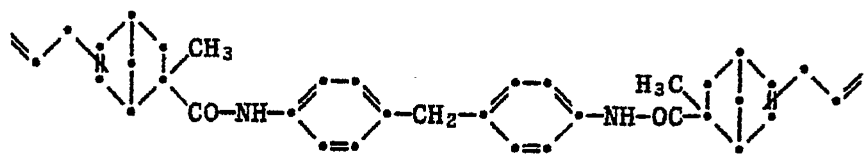

53,0 g 4,4'-Diaminodiphenylmethan und 113 g Allyl-bicyclo [2.2.1]hept-5-en-2-methyl-2-carbonylchlorid werden nach Verfahren A umgesetzt. Man isoliert ein rotbraunes Festharz mit einem Erweichungspunkt von 40°C. Durch Gelpermeationschromatographie (THF) ermittelt man ein Molekulargewicht von 674 ($\bar{M}_n$) bzw. 714 ($\bar{M}_w$). Im IR-Spektrum ist eine Absorptionsbande bei 3340 $cm^{-1}$ (-NH-) bzw. bei 1660 $cm^{-1}$ 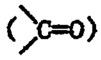. vorhanden.

| Analyse | %C | %H | %N |
|---|---|---|---|
| berechnet für $C_{37}H_{42}N_2O_2$ | 81,28 | 7,74 | 5,12 |
| gefunden | 80,52 | 7,82 | 4,76 |

Beispiel 28: Herstellung des Bisamids aus 3-Aminomethyl-3,5,5-trimethylcyclohexylamin und Allyl-bicyclo [2.2.1]hept-5-en-2-carbonsäure

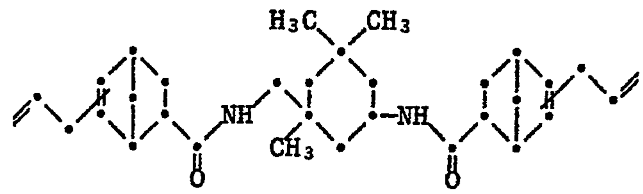

7,8 g 3-Aminomethyl-3,5,5-trimethylcyclohexyl-amin und 19,6 g Allyl-bicyclo[2.2.1]hept-5-en-2-carbonylchlorid werden nach Verfahren A umgesetzt. Man erhält 19,4 g (79 % der Theorie) eines hellbraunen Festharzes, mit einem Erweichungspunkt von 63°C. Durch Gelpermeationschromatographie (THF) ermittelt man ein Molekulargewicht von 628 ($\bar{M}_n$) bzw. von 1025 ($\bar{M}_w$). Das IR-Spektrum zeigt eine Absorptionsbande bei 3310 $cm^{-1}$ (-NH-) bzw. bei 1650 $cm^{-1}$ 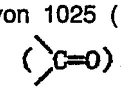.

| Analyse | %C | %H | %N |
|---|---|---|---|
| berechnet für $C_{32}H_{46}N_2O_2$ | 78,32 | 9,45 | 5,71 |
| gefunden | 72,26 | 9,49 | 5,71 |

Beispiel 29: Herstellung von N,N',N'',N'''-Triethylen-tetra-(allyl-bicyclo[2.2.1]hept-5-en-2-carbonsäureamid)

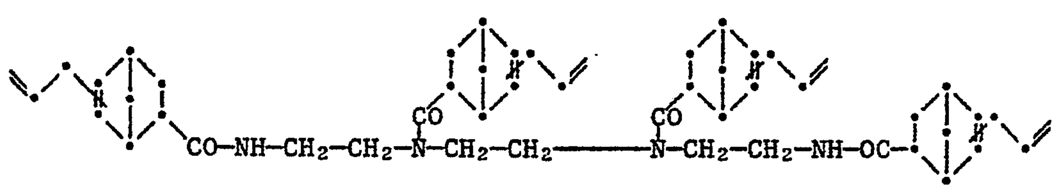

13,9 g Triethylentetramin und 75,0 g Allyl-bicyclo [2.2.1]hept-5-en-2-carbonylchlorid werden nach Verfahren A umgesetzt. Man erhält 54,0 g (72 % der Theorie) eines rotbraunen Festharzes mit einem Erweichungspunkt von 82°C und einem durch Gelpermeationschromatographie (THF) ermittelten Mole-

kulargewicht von 898 ($\bar{M}_n$) bzw. 1561 ($\bar{M}_w$). Das IR-Spektrum zeigt eine Absorptionsbande bei 3320 $cm^{-1}$ (-NH-) bzw. 1640 $cm^{-1}$ ( 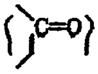 ).

| Analyse | | | |
|---|---|---|---|
| | % C | % H | % N |
| berechnet für $C_{50}H_{66}N_4O_4$ | 76,30 | 8,45 | 7,12 |
| gefunden | 75,17 | 8,37 | 7,15 |

Herstellung von vernetzten Polymeren

Die erfindungsgemässen Verbindungen lassen sich thermisch polymerisieren und man erhält vernetzte Polymere mit wertvollen physikalischen Eigenschaften.

Beispiele:

I. Das gemäss Beispiel 21 hergestellte Diamid giesst man als heisses, dünnflüssiges Harz in eine Stahlform von 12 x 12 x 0,4 $cm^3$ und härtet 16 Stunden bei 200°C aus. Nach dem Abkühlen schneidet man Prüfstäbe aus der klaren, rotbraunen Platte. Folgende Eigenschaften werden daran gemessen:
Biegefestigkeit nach ISO 178 (Messung bei 23°C) = 79 $N/mm^2$
Schlagbiegefestigkeit nach VSM 77105 = 8,3 $kJ/m^2$
Formbeständigkeit nach ISO 75 = 226°C
Glasumwandlungstemperatur $T_g$ (gemessen mit TA 2000[1]) = 235°C
Wasseraufnahme (1 Stunde 100°C) = 0,26 %
10 % Gewichtsverlust[2] = 408°C

Beim Verkleben von Aluminiumblech unter den gleichen Härtungsbedingungen, wobei die beiden zu verklebenden Bleche sich mit 25 x 12 $mm^2$ überlappen, erhält man eine Zugscherfestigkeit nach ISO 4587 (Messung bei Raumtemperatur) von 9,1 $N/mm^2$.

[1] TA 2000 = Differentialthermoanalysesystem TA 2000 der Firma Mettler AG, Greifensee, CH.
[2] Messung durch Aufheizen einer Probe in TA 2000; Ermittlung der Temperatur, bei der 10 % der Probe verflüchtigt sind; Aufheizgeschwindigkeit: 4°C/min. (in Luft).

II. Das gemäss Beispiel 20A hergestellte Diamid wird in eine Stahlform von 12 x 12 x 0,4 $cm^3$ gegossen und während 4 Stunden bei 160°C und 10 Stunden bei 200°C gehärtet. Folgende Eigenschaften werden gemessen:
Biegefestigkeit (ISO 178) = 130 $N/mm^2$
Schlagbiegefestigkeit (VSM 77105) = 11,9 $kJ/m^2$
Formbeständigkeit (ISO 75) = 174°C
Glasumwandlungstemperatur $T_g$ = 187°C
Wasseraufnahme (1 Stunde bei 100°C) = 0,41 %
10 % Gewichtsverlust = 392°C
Zugscherfestigkeit (ISO 4587) = 10,0 $N/mm^2$

III. Das gemäss Beispiel 20B hergestellt Diamid wird in eine Stahlform von 8 x 6 x 0,4 $cm^2$ gegossen und 16 Stunden bei 200°C und 7 Stunden bei 220°C gehärtet.
Schlagbiegefestigkeit (VSM 77105) = 9,5 $kJ/m^2$
Glasumwandlungstemperatur $T_g$ = 134°C
10 % Gewichtsverlust = 423°C

IV. Das gemäss Beispiel 22 hergestellte Diamid wird in eine Stahlform von 8 x 6 x 0,4 $cm^3$ gegossen und wie in Beispiel II gehärtet.
Schlagbiegefestigkeit (VSM 77105) = 7,3 $kJ/m^2$
Glasumwandlungstemperatur $T_g$ = 278°C
Zugscherfestigkeit (ISO 4587) = 12,9 $N/mm^2$

V. Das gemäss Beispiel 24 hergestellte Diamid wird in eine Stahlform von 12 x 12 x 0,4 $cm^3$ gegossen und wie in Beispiel II gehärtet.
Biegefestigkeit (ISO 178) = 71 $N/mm^2$
Schlagbiegefestigkeit (VSM 77105) = 4,6 $kJ/m^2$
Formbeständigkeit (ISO 75) = > 250°C
Glasumwandlungstemperatur $T_g$ = 256°C
Wasseraufnahme (1 Stunde bei 100°C) = 0,56 %
10 % Gewichtsverlust = 403°C

VI. Das gemäss Beispiel 25 hergestellte Diamid wird in eine Stahlform von 8 x 6 x 0,4 $cm^3$ gegossen und wie in Beispiel II gehärtet.
Schlagbiegefestigkeit (VSM 77105) = 6,8 $kJ/m^2$
Glasumwandlungstemperatur $T_g$ = 265°C
10 % Gewichtsverlust = 409°C

VII. Die Polymerisation des gemäss Beispiel 26 hergestellten Diamids während 4 Stunden bei 160°C, 2 Stunden bei 180°C und 10 Stunden bei 200°C ergibt einen klaren, roten Festkörper mit einem $T_g$ von 256°C.

VIII. Das gemäss Beispiel 27 hergestellte Diamid wird in eine Stahlform von 8 x 6 x 0,4 $cm^3$ gegossen und wie in Beispiel II gehärtet.
Schlagbiegefestigkeit (VSM 77105) = 2,1 $kJ/m^2$
Glasumwandlungstemperatur $T_g$ = 213°C

IX. Das gemäss Beispiel 28 hergestellte Diamid wird in eine Stahlform von 8 x 6 x 0,4 $cm^3$ gegossen und wie in Beispiel II gehärtet.
Schlagbiegefestigkeit (VSM 77105) = 6,1 $kJ/m^2$
Glasumwandlungstemperatur $T_g$ = 248°C
10 % Gewichtsverlust = 408°C
Zugscherfestigkeit (ISO 4587) = 6,7 $N/mm^2$

X. Das gemäss Beispiel 29 hergestellte Diamid wird in eine Stahlform von 8 x 6 x 0,4 $cm^3$ gegossen und wie in Beispiel II gehärtet.
Schlagbiegefestigkeit (VSM 77105) = 5,2 $kJ/m^2$
Glasumwandlungstemperatur $T_g$ = 286°C
10 % Gewichtsverlust = 402°C

XI. Unter Rühren löst man 56 g des gemäss Beispiel 24 hergestellten Diamids in 100 g Methylenchlorid. Die klare, gelbe Lösung verwendet man als Imprägnierlösung zur Herstellung von Prepregs.

Prepreg-Herstellung: Gewebebahnen aus Kohlenstoff-Fasern vom Typ G 814 NT der Fa. Brochier S.A. mit den Abmessungen 245 x 16 cm werden durch die oben beschriebenen Imprägnierlösungen gezogen und während 10 Minuten zum Abtropfen aufgehängt. Anschliessend werden sie auf ein Trockengestell aufgezogen und während 8 Minuten bei 160°C in einem Umluftofen getrocknet. Die getrockneten C-Fasergewebebahnen werden in 13,5 x 14,5 cm grosse Stücke zerschnitten. Der Harzgehalt der Prepregs beträgt gemäss gravimetrischer Messung 49 %.

Laminat-Herstellung: Jeweils 11 Lagen Prepreg (13,5 x 14,5 cm) werden auf beiden Seiten mit einer Kupferfolie abgedeckt und in eine Poly(diphenyloxid-pyromellitimid)-Folie (Kapton® der Fa. Du Pont) eingewickelt. Die Prepregs werden dann in eine auf 180°C vorgeheizte Presse gegeben. Nach Kontaktzeit von 2 Minuten wird im Verlauf von 2 Minuten ein Druck von 907 kg aufgepresst, wodurch ein ausreichender Fluss der Harzmischung gewährleistet bleibt. Nach 7 Minuten wird der Druck innerhalb von 3 Minuten auf 3628 kg erhöht, und dieser Druck wird während 1 Std. bei 180°C aufrechterhalten. Das so erhaltene Laminat wird abgekühlt und bei 100-120°C aus der Presse entfernt. Man erhält ein Laminat mit sehr guter Oberfläche ohne sichtbare Leerstellen. Das Laminat wird anschliessend wie folgt in einem Umluftofen nachgehärtet: 6 Stunden bei 200°C.
Nach der Härtung erhält man ein qualitativ hochwertiges Laminat mit vertvollen mechanischen und physikalischen Eigenschaften.

## Patentansprüche

1. Verbindungen der Formel I

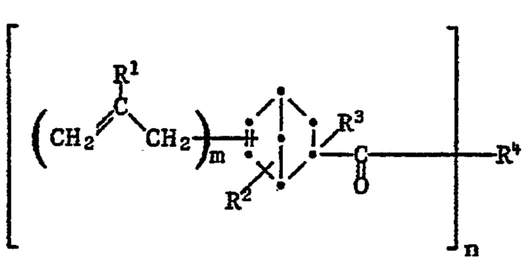

(I),

worin $R^1$, $R^2$ und $R^3$ unabhängig voneinander Wasserstoff oder Methyl bedeuten,
m eine ganze Zahl von 1 bis 5 ist,
n eine ganze Zahl von 1 bis 5 bedeutet, und
$R^4$ $-NH_2$ oder ein n-wertiger Rest eines primären und/oder sekundären aliphatischen, cycloaliphatischen, aromatischen, araliphatischen oder heterocyclischen (Poly)amins nach dem Entfernen von n Aminwasserstoffatomen ist, der über die Aminogruppen an die jeweiligen Carbonylreste gebunden ist.

2. Verbindungen der Formel I gemäss Anspruch 1, worin $R^1$, $R^2$ und $R^3$ Wasserstoff sind.

3. Verbindungen der Formel I gemäss Anspruch 1, worin n eine ganze Zahl von 2 bis 5 ist, und $R^4$ sich ableitet von α,ω-Alkylendiaminen, m- oder p-Phenylendiamin, m- oder p-Xylylendiamin, sowie von Verbindungen der Formeln III, IV, V, VI oder VII

$$R^9HN\text{-}(\text{-}Z\text{-}O\text{-})_p\text{-}Z\text{-}NHR^9 \qquad \text{(III)},$$

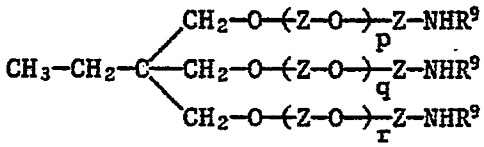

(IV),

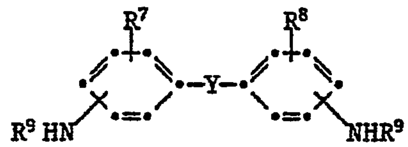

(V),

$$R^9HN(\text{-}CH_2\text{-}CH_2\text{-}NH\text{-})_o\text{-}CH_2\text{-}CH_2\text{-}NHR^9 \qquad \text{(VI)},$$

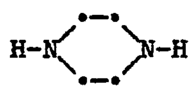

(VII),

worin die Brücke Y eine direkte C-C-Bindung ist, oder -O-, -S-, $-SO_2-$, -CO-, $-CH_2-$ oder $-C(CH_3)_2-$ bedeutet, $R^7$, $R^8$ und $R^9$ unabhängig voneinander Wasserstoff oder $C_1$-$C_5$Alkyl sind, Z einer der Reste $-CH_2\text{-}CH_2-$, $-CH_2\text{-}CH_2\text{-}CH_2-$ oder $-CH_2\text{-}CH(CH_3)-$ ist, der Index o eine ganze Zahl von 0 bis 2 ist und die Indizes p, q und r unabhängig voneinander ganze Zahlen von 1 bis 8 bedeuten.

4. Verbindungen der Formel I gemäss Anspruch 1, worin m 1 oder 2 ist und n 2 bedeutet, $R^4$ ausgewählt wird aus der Gruppe bestehend aus

$-R^{10}N\text{-}(CH_2)_u\text{-}NR^{10}-$, 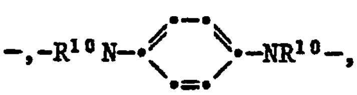, 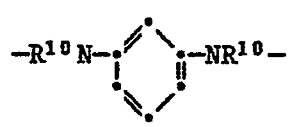 und

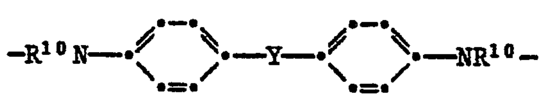,

worin Y -O-, $-SO_2-$, -CO- oder $-CH_2-$ ist, $R^{10}$ Wasserstoff, $C_1$-$C_5$Alkyl oder Phenyl ist und u eine ganze Zahl von 2 bis 12 bedeutet.

5. Verbindungen der Formel I gemäss Anspruch 1, worin $R^1$ und $R^2$ Wasserstoff sind und $R^3$ Methyl darstellt.

6. Die Verbindungen

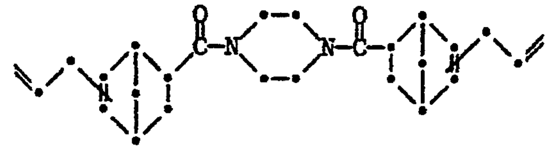

und

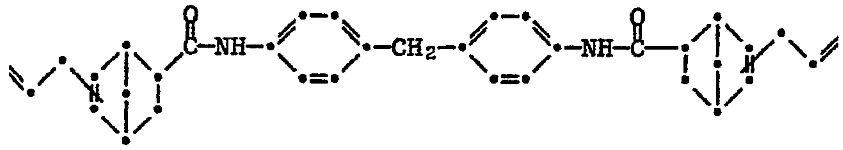

gemäss Anspruch 1.

7. Verbindungen der Formel I gemäss Anspruch 1, worin m 1 oder 2 ist, n 1 ist, $R^4$ $-NR^{12}R^{13}$ ist und $R^{12}$ und $R^{13}$ unabhängig voneinander Wasserstoff, $C_1$-$C_8$Alkyl, Allyl, Cyclohexyl, Phenyl, Benzyl oder Tolyl bedeuten.

8. Verbindungen der Formel VIII

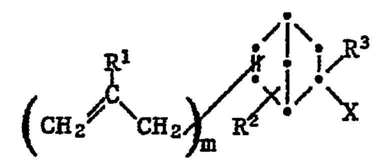

(VIII),

worin $R^1$, $R^2$ und $R^3$ unabhängig voneinander Wasserstoff oder Methyl bedeuten.
X -COOH, $-COOR^{11}$, -COCl, -COBr, -CHO oder -CN ist,
$R^{11}$ $C_1$-$C_{20}$Alkyl, $C_3$-$C_{12}$Alkenylmethyl, $C_3$-$C_{12}$Alkinylmethyl, $C_3$-$C_{12}$Cycloalkyl, Phenyl, Naphthyl, $C_7$-$C_9$Aralkyl, $C_7$-$C_9$Alkaryl oder einen fünf- bis sechsgliedrigen heterocyclischen Ring mit ein bis drei Sauerstoff-, Schwefel- und/oder Stickstoffatomen darstellt und m eine ganze Zahl von 1 bis 5 bedeutet.

9. Verbindungen der Formel VIII gemäss Anspruch 8, worin X -COOH, $-COOR^{11}$ oder -COCl bedeutet,
$R^{11}$ $C_1$-$C_8$Alkyl, Allyl, Cyclohexyl, Phenyl, Benzyl, Tolyl oder Furan-2-yl darstellt und
m 1 oder 2 ist.

10. Verbindungen der Formel VIII gemäss Anspruch 9, worin $R^1$, $R^2$ und $R^3$ Wasserstoff sind, X einen der Reste -COOH, $-COOCH_3$, $-COOC_6H_5$ oder -COCl darstellt und m 1 oder 2 bedeutet.

11. Verfahren zur Herstellung von Verbindungen der Formel I gemäss Anspruch 1 durch Umsetzung eines Carbonsäurechlorids der Formel IX

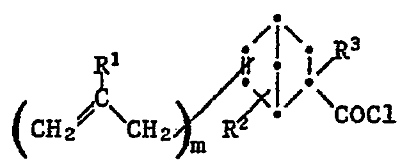

(IX),

worin $R^1$, $R^2$ und $R^3$ unabhängig voneinander Wasserstoff oder Methyl bedeuten und m eine ganze Zahl von 1 bis 5 ist, mit einem primären und/oder sekundären aliphatischen, cycloaliphatischen, aromatischen, araliphatischen oder heterocyclischen (Poly)amin mit ein bis fünf Aminogruppen, wobei die Aminogruppen des (Poly)amins in mindestens der dem Carbonsäurechlorid äquivalenten Menge vorliegen müssen, in Gegenwart von mindestens der dem Carbonsäurechlorid äquivalenten Menge eines tertiären Amins, oder
durch Umsetzung eines Carbonsäurechlorids der Formel IX, wie oben definiert, mit mindestens der zweifach äquivalenten Menge an Aminogruppen eines (Poly)amins, wie oben definiert.

12. Verfahren zur Herstellung von Verbindungen der Formel I mit einem aliphatischen, cycloaliphatischen oder araliphatischen Rest $R^4$ gemäss Anspruch 1 durch Umsetzung eines Carbonsäureesters der Formel X

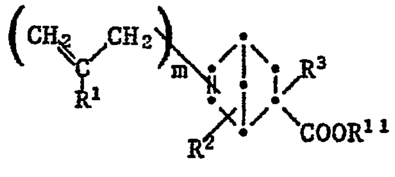

(X),

worin $R^1$, $R^2$, $R^3$, $R^{11}$ und m die in Anspruch 8 definierte Bedeutung besitzen, mit einem primären aliphatischen, cycloaliphatischen oder araliphatischen (Poly)amin mit ein bis fünf Aminogruppen, wobei die Aminogruppen des (Poly)amins in etwa der 1,1- bis 2,0-fachen molaren Menge, bezogen auf die Menge des Carbonsäureesters X, vorliegen, in Gegenwart eines basischen Katalysators bei Temperaturen zwischen 120 und 160°C.

13. Polymere, die dadurch erhältlich sind, dass man eine Verbindung der Formel I gemäss Anspruch 1 während 6 bis 60 Stunden auf eine Temperatur zwischen 150 und 300°C erhitzt.

14. Verwendung der Verbindungen der Formel I gemäss Anspruch 1 zur Herstellung gehärteter Produkte.

## Claims

1. A compound of formula I

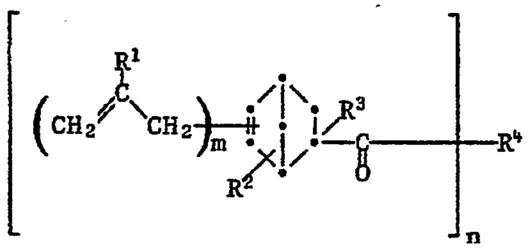

(I)

wherein
$R^1$, $R^2$ and $R^3$ are each independently of the other hydrogen or methyl,
m is an integer from 1 to 5,
n is an integer from 1 to 5, and
$R^4$ is $-NH_2$ or an n-valent radical of a primary and/or secondary aliphatic, cycloaliphatic, aromatic, araliphatic or heterocyclic (poly)amine after removal of n amine hydrogen atoms, which radical is attached to the respective carbonyl radicals through the amino groups.

2. A compound of formula I according to claim 1, wherein $R^1$, $R^2$ and $R^3$ are hydrogen.

3. A compound of formula I according to claim 1, wherein n is an integer from 2 to 5, and $R^4$ is derived from α,ω-alkylenediamines, m- or p-phenylenediamine, m- or p-xylylenediamine, and from compounds of formula III, IV, V, VI or VII

$$R^9HN\text{-}(\text{-}Z\text{-}O\text{-})_p\text{-}Z\text{-}NHR^9$$ (III),

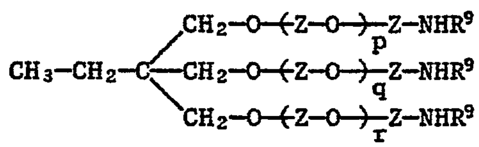

(IV),

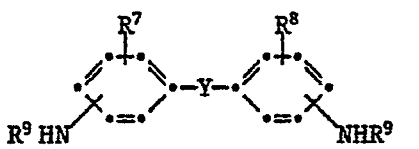

(V),

$$R^9HN(CH_2\text{-}CH_2\text{-}NH)_o\text{-}CH_2\text{-}CH_2\text{-}NHR^9$$ (VI),

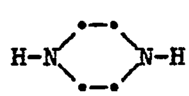

(VII),

in which formulae the bridge Y is a direct C–C bond, or is $-O-$, $-S-$, $-SO_2-$, $-CO-$, $-CH_2-$ or $-C(CH_3)_2-$, $R^7$, $R^8$ and $R^9$ are each independently of the other hydrogen or $C_1$–$C_5$alkyl, Z is one of the radicals $-CH_2-CH_2-$, $-CH_2-CH_2-CH_2-$ or $-CH_2-CH(CH_3)-$, the index o is an integer from 0 to 2 and the indices p, q and r are each independently of the other integers from 1 to 8.

4. A compound of formula I according to claim 1, wherein m is 1 or 2 and n is 2, $R^4$ is selected from the group consisting of

$-R^{10}N-(CH_2)_u-NR^{10}-$, 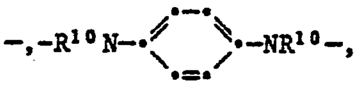, 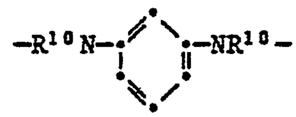 and

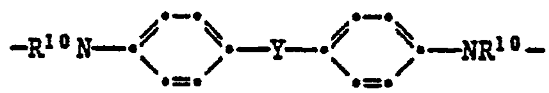 ,

in which formulae Y is $-O-$, $-SO_2-$, $-CO-$ or $-CH_2-$, $R^{10}$ is hydrogen, $C_1$–$C_5$alkyl or phenyl and u is an integer from 2 to 12.

5. A compound of formula I according to claim 1, wherein $R^1$ and $R^2$ are hydrogen and $R^3$ is methyl.

6. The compounds

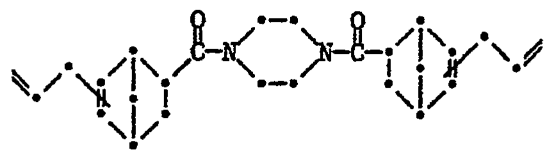

and

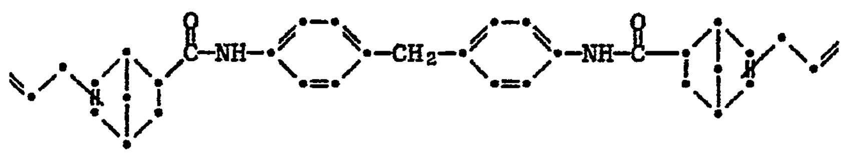

according to claim 1.

7. A compound of formula I according to claim 1, wherein m is 1 or 2, n is 1, $R^4$ is $-NR^{12}R^{13}$ and $R^{12}$ and $R^{13}$ are each independently of the other hydrogen, $C_1$–$C_8$alkyl, allyl, cycylohexyl, phenyl, benzyl or tolyl.

8. A compound of formula VIII

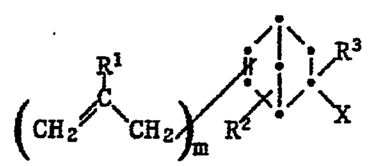 (VIII),

wherein
$R^1$, $R^2$ and $R^3$ are each independently of the other hydrogen or methyl,
X is $-COOH$, $-COOR^{11}$, $-COCl$, $-COBr$, $-CHO$ or $-CN$,
$R^{11}$ is $C_1$–$C_{20}$alkyl,
$C_3$–$C_{12}$alkenylmethyl, $C_3$–$C_{12}$alkynylmethyl, $C_3$–$C_{12}$cycloalkyl, phenyl, naphthyl, $C_7$–$C_9$aralkyl, $C_7$–$C_9$alkaryl or a five- or six-membered heterocyclic ring containing one to three oxygen, sulfur and/or nitrogen atoms and
m is an integer from 1 to 5.

9. A compound of formula VIII according to claim 8, wherein
X is $-COOH$, $-COOR^{11}$ or $-COCl$,
$R^{11}$ is $C_1$–$C_8$alkyl, allyl, cyclohexyl, phenyl, benzyl, tolyl or furan-2-yl and
m is 1 or 2.

10. A compound of formula VIII according to claim 9, wherein $R^1$, $R^2$ and $R^3$ are hydrogen, X is one of the radicals $-COOH$, $-COOCH_3$, $-COOC_6H_5$ or $-COCl$ and m is 1 or 2.

11. Process for the preparation of compounds of formula I according to claim 1 by reacting a carboxylic acid chloride of formula IX

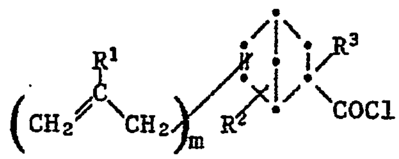

(IX),

wherein $R^1$, $R^2$ and $R^3$ are each independently of the other hydrogen or methyl and m is an integer from 1 to 5, with a primary and/or secondary aliphatic, cycloaliphatic, aromatic, araliphatic or heterocyclic (poly)amine containing one to five amino groups, which amino groups of the (poly)amine must be present in an amount at least equivalent to the amount of carboxylic acid chloride, in the presence of a tertiary amine in an amount at least equivalent to the amount of carboxylic acid chloride, or by reacting a carboxylic acid chloride of formula IX as defined above with at least twice the equivalent amount of amino groups of a (poly)amine as defined above.

12. A process for the preparation of compounds of formula I containing an aliphatic, cycloaliphatic or araliphatic radical $R^4$ according to claim 1 by reacting a carboxylic acid ester of formula X

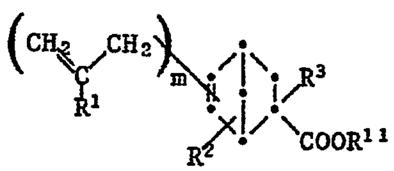

(X),

wherein $R^1$, $R^2$, $R^3$, $R^{11}$ and m are as defined in claim 8, with a primary aliphatic, cycloaliphatic or araliphatic (poly)amine containing one to five amino groups, which amino groups of the (poly)amine are present in about 1.1 to 2.0 times the molar amount, based on the amount of the carboxylic acid ester X, in the presence of a basic catalyst in the temperature range from 120 to 160°C.

13. A polymer which is obtainable by heating a compound of formula I according to claim 1 for 6 to 60 hours at a temperature in the range from 150 to 300°C.

14. Use of the compounds of formula I according to claim 1 for the preparation of cured products.

## Revendications

1. Composés répondant à la formule I

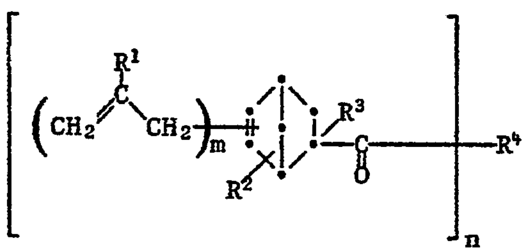

(I)

dans laquelle

$R^1$, $R^2$ et $R^3$ représentent chacun, indépendamment les uns des autres, l'hydrogène ou un radical méthyle,

m désigne un nombre entier de 1 à 5,

n désigne un nombre entier de 1 à 5, et

$R^4$ représente un radical $-NH_2$ ou le radical de valence n qui subsiste après qu'on a éliminé n atomes d'hydrogène aminés d'une amine ou polyamine primaire et/ou secondaire, aliphatique, cycloaliphatique, aromatique, araliphatique ou hétérocyclique et qui est lié par les radicaux amino aux radicaux carbonyles correspondants.

2. Composés de formule I selon la revendication 1 dans lesquels $R^1$, $R^2$ et $R^3$ représentent chacun l'hydrogène.

3. Composés selon la revendication 1 dans lesquels n représente un nombre entier de 2 à 5, et $R^4$ dérive d'une α,ω-alkylène-diamine, de la m- ou p-phénylène-diamine, de la m- ou p-xylylène-diamine, ou d'un composé répondant à l'une des formules III, IV, V, VI et VII suivantes:

$R^9HN{-}({-}Z{-}O{-})_p{-}Z{-}NHR^9$ (III),

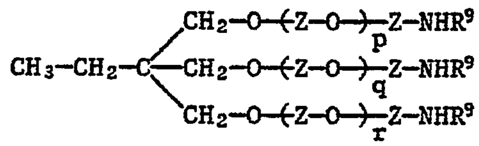

(IV),

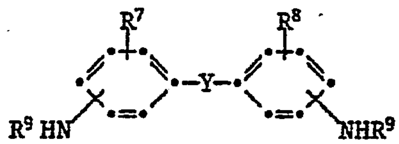

(V),

$R^9HN({-}CH_2{-}CH_2{-}NH{-})_o CH_2{-}CH_2{-}NHR^9$ (VI),

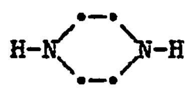

(VII),

formules dans lesquelles le pont Y représente une liaison directe carbone-carbone ou un radical –O–, –S–, $-SO_2-$, –CO–, $-CH_2-$ ou $-C(CH_3)_2-$, $R^7$, $R^8$ et $R^9$ représentent chacun, indépendamment les uns des autres, l'hydrogène ou un alkyle en $C_1$–$C_5$, Z représente un radical $-CH_2-CH_2-$, $-CH_2-CH_2-CH_2-$ ou $-CH_2-CH(CH_3)-$, l'indice ou désigne un nombre entier de 0 à 2, et les indices p, q et r représentent chacun, indépendamment les uns des autres, un nombre entier de 1 à 8.

4. Composés de formule I selon la revendication 1 dans lesquels m est égal à 1 ou à 2, n est égal à 2 et $R^4$ est choisi dans l'ensemble constitué par les radicaux répondant aux formules suivantes:

$-R^{10}N{-}({-}CH_2{-})_u{-}NR^{10}-$, 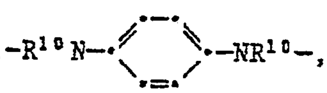, 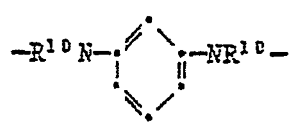 et

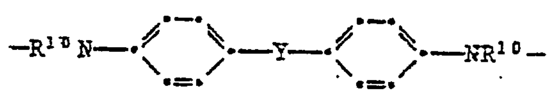

,

dans lesquelles Y représente –O–, $-SO_2-$, –CO– ou $-CH_2-$, $R^{10}$ représente l'hydrogène, un alkyle en $C_1$–$C_5$ ou un phényle et ou désigne un nombre entier de 2 à 12.

5. Composés de formule I selon la revendication 1 dans lesquels $R^1$ et $R^2$ représentent chacun l'hydrogène et $R^3$ représente un méthyle.

6. Composés selon la revendication 1, en l'espèce celui qui répond à la formule:

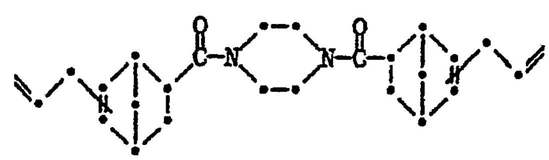

et celui qui répond à la formule:

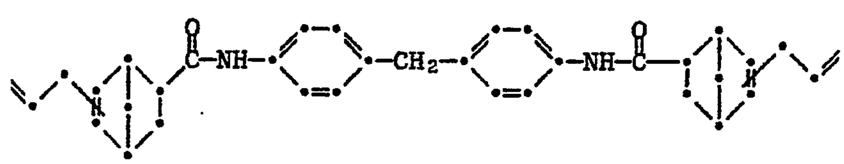

7. Composés de formule I selon la revendication 1 dans lesquels m est égal à 1 ou à 2, n est égal à 1 et $R^4$ représente un radical $-NR^{12}R^{13}$ dans lequel les symboles $R^{12}$ et $R^{13}$ représentent chacun, indépen-

damment l'un de l'autre, l'hydrogène, un alkyle en $C_1$ à $C_8$, un allyle, un cyclohexyle, un phényle, un benzyle ou un tolyle.

8. Composés répondant à la formule VIII:

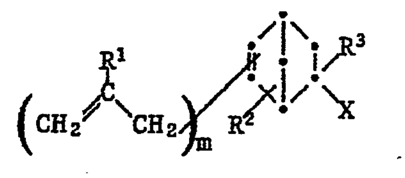

(VIII),

dans laquelle
$R^1$, $R^2$ et $R^3$ représentent chacun, indépendamment les uns des autres, l'hydrogène ou un méthyle,
X représente un radical $-COOH$, $-COOR^{11}$, $-COCl$, $-COBr$, $-CHO$ ou $-CN$,
le symbole $R^{11}$ désignant un alkyle en $C_1$ à $C_{20}$, un alcénylméthyle en $C_3$–$C_{12}$, un alcynylméthyle en $C_3$–$C_{12}$, un cycloalkyle en $C_3$–$C_{12}$, un phényle, un naphtyle, un aralkyle en $C_7$–$C_9$, un alkylaryle en $C_7$–$C_9$ ou un radical hétérocyclique à 5 ou 6 maillons qui contient de 1 à 3 atomes d'oxygène, de soufre et/ou d'azote, et
m désigne un nombre entier de 1 à 5.

9. Composés de formule VIII selon la revendication 8 dans lesquels X représente un radical $-COOH$, $-COOR^{11}$ ou $-COCl$, $R^{11}$ représente un alkyle en $C_1$–$C_8$, un allyle, un cyclohexyle, un phényle, un benzyle, un tolyle ou un furannyle-2 et m est égal à 1 ou à 2.

10. Composés de formule VIII selon la revendication 9 dans lesquels $R^1$, $R^2$ et $R^3$ représentent chacun l'hydrogène, X représente un radical $-COOH$, $-COOCH_3$, $-COOC_6H_5$ ou $-COCl$ et m est égal à 1 ou à 2.

11. Procédé de préparation de composés de formule I selon la revendication 1, procédé selon lequel on fait réagir un chlorure d'acide carboxylique répondant à la formule IX

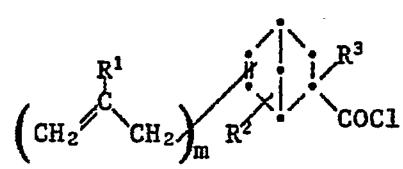

(IX),

dans laquelle $R^1$, $R^2$ et $R^3$ représentent chacun, indépendamment les uns des autres, l'hydrogène ou un radical méthyle et m désigne un nombre entier de 1 à 5, avec une amine ou polyamine primaire et/ou secondaire, aliphatique, cycloaliphatique, aromatique, araliphatique ou hétérocyclique qui contient de 1 à 5 radicaux amino, les radicaux amino de l'amine ou de la polyamine devant se trouver en une quantité au moins équivalente à celle du chlorure d'acide carboxylique, en présence d'une quantité d'une amine tertiaire au moins équivalente à celle du chlorure d'acide carboxylique, ou on fait réagir un chlorure d'acide carboxylique de formule IX tel que défini ci-dessus, avec une quantité au moins double de la quantité équivalente de radicaux amino d'une amine ou polyamine telle que définie ci-dessus.

12. Procédé pour préparer des composés de formule I dans lesquels $R^4$ représente un radical aliphatique, cycloaliphatique ou araliphatique, procédé selon lequel on fait réagir un ester d'acide carboxylique répondant à la formule X:

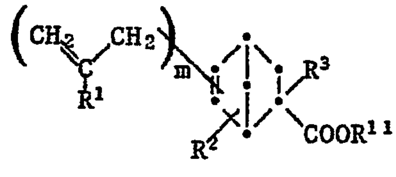

(X),

dans laquelle $R^1$, $R^2$, $R^3$, $R^{11}$ et m ont les significations qui leur ont été données à la revendication 8, avec une amine ou polyamine primaire aliphatique, cycloaliphatique ou araliphatique contenant de 1 à 5 radicaux amino, les radicaux amino de l'amine ou de la polyamine se trouvant en une quantité comprise environ entre 1,1 et 2,0 fois la quantité molaire relativement à la quantité de l'ester d'acide carboxylique de formule X, en présence d'un catalyseur basique à une température comprise entre 120 et 160°C.

13. Polymères que l'on peut obtenir en chauffant un composé de formule I selon la revendication 1 pendant 6 à 60 heures à une température comprise entre 150 et 300°C.

14. Application des composés de formule I selon la revendication 1 à la fabrication de produits durcis.